# EUROPEAN PATENT APPLICATION

(11) **EP 3 851 438 A1**
(43) Date of publication of application: **21.07.2021**
(21) Application number: 20773247.0
(22) Date of filing: 18.03.2020
(51) Int. Cl.: C07D 471/04, A61K 31/437, A61P 35/00

(54) **BTK INHIBITOR, PHARMACEUTICALLY ACCEPTABLE SALT, POLYMORPH AND APPLICATION THEREOF**

(30) Priority: 18.03.2019 CN 201910203239
(71) Applicant: Shanghai Haiyan Pharmaceutical Technology Co., Ltd, Shanghai 201203 (CN); Yangtze River Pharmaceutical Group Co., Ltd., Taizhou, Jiangsu 225321 (CN)
(72) Inventor: JIANG, Taotao, Shanghai 201203 (CN); LI, Jinjing, Shanghai 201203 (CN); ZHAO, Shuangni, Shanghai 201203 (CN); YAO, Xia, Shanghai 201203 (CN)
(74) Representative: Coles, Andrea Birgit
(86) International application number: PCT/CN2020/080024
(87) International publication number: WO 2020/187267

(57) **Abstract**

The present invention provides a BTK inhibitor, a pharmaceutically acceptable salt, a polymorph and an application thereof. Specifically, the present invention provides (R)-6-((1-acryloylpiperidin-3-yl)amino)-7-fluoro-4-((2-fluoro-4-morpholinophenyl)amino)-1,2-dihydro-3H-pyrrolo[3,4-c]pyridin-3-one, or the polymorph of a pharmaceutically acceptable salt thereof, and an application thereof. In addition, the present invention further discloses a pharmaceutical composition comprising the inhibitor and an application thereof.

## Description

### TECHNICAL FIELD

The present disclosure belongs to the technical field of medicine. In particular, the present disclosure relates to a BTK inhibitor, a pharmaceutically acceptable salt and polymorph thereof, and their applications, and the inhibitor is (R)-6-((1-acryloylpiperidin-3 -yl)amino)-7-fluoro-4-((2-fluoro-4-morpholinophenyl)amino)-1,2-dihydrogen-3H-pyrrolo[3,4-c]p yridin-3-one.

### BACKGROUND

BTK kinase, a non-receptor tyrosine kinase of the TEC kinase family and a key regulator of the BCR signaling pathway, plays an important role in B cell maturation, proliferation and survival. BTK is overexpressed in a variety of B-cell lymphomas and is the only clinically proven effective target for drug development in the TEC kinase family. Inhibition of BTK can inhibit proliferation of a range of B cell lymphomas.

Activation of B cell antigen receptor (BCR) signaling pathway plays an important role in inducing and maintaining B cell malignancies and autoimmune diseases. Bruton's tyrosine kinase (Btk) plays a key role in the hematopoietic cell BCR signaling pathway and is a very good target for new lymphoma therapy. BTK inhibitors act on the BCR pathway, inhibit Btk autophosphorylation, phosphorylation of Btk's physiological substrate PLCγ and phosphorylation of the downstream kinase ERK.

BTK inhibitors act on chronic lymphocytic leukemia (CLL) cells, induce cytotoxicity, and inhibit the proliferation of CLL cells. It inhibits the proliferation of primary B cells activated by BCR and the secretion of TNFα, IL-1β,IL-6 and the like in primary monocytes. BTK inhibitors act on collagen-induced arthritis models and significantly reduce clinical arthritis symptoms, such as foot swelling, joint inflammation, etc., by inhibiting B cell activity.

Currently, only one BTK inhibitor, ibrutinib, has been approved for marketing, so it is necessary to develop more active, safer and more effective BTK inhibitors. The present disclosure develops various salt forms and crystalline forms of the BTK inhibitor on the basis of the foregoing work, which is helpful for further drug development.

### SUMMARY OF THE INVENTION

An object of the present disclosure is to provide a pharmaceutically acceptable salt of a BTK inhibitor, a polymorph thereof, and their applications.

In a first aspect of the present disclosure, the compound of formula X, a pharmaceutically acceptable salt of the compound of formula X, a polymorph of the compound of formula X or a polymorph of pharmaceutically acceptable salt of the compound of formula X is provided:

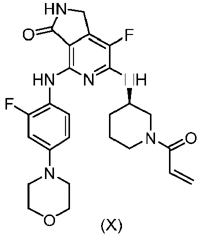

In one embodiment, the pharmaceutically acceptable salt is selected from the group consisting of hydrochloride, sulfate, hydrobromide, phosphate, methanesulfonate, maleate, L-tartrate, citrate, fumarate, and succinate.

In one embodiment, the pharmaceutically acceptable salt of the compound of formula X, the polymorph of the compound of formula X and the polymorph of pharmaceutically acceptable salt of the compound of formula X is in an anhydrous form, hydrate form or solvate form.

In one embodiment, the pharmaceutically acceptable salt is selected from the group consisting of hydrochloride, sulfate, hydrobromide, phosphate, methanesulfonate, L-tartrate, fumarate, and succinate.

In one embodiment, the pharmaceutically acceptable salt is hydrochloride, and the molar ratio of hydrochloric acid to the compound of formula X is (0.8-2.1): 1, for example (0.9-1.1): 1.

In one embodiment, the pharmaceutically acceptable salt is fumarate, and the molar ratio of fumaric acid to the compound of formula X is (0.8-1.2):1. In another embodiment, the pharmaceutically acceptable salt is fumarate, and the molar ratio of fumaric acid to the compound of formula X is (0.9-1.1):1. In another embodiment, the pharmaceutically acceptable salt is fumarate, and the molar ratio of fumaric acid to the compound of formula X is 1:1.

In one embodiment, the polymorph is A crystalline form of the hydrochloride of compound of formula X, i.e. crystal form A, the X-ray powder diffraction pattern of which has peaks at diffraction angles 2θ(°) values of the following group A-1:14.75±0.2, 15.97±0.2, 17.20±0.2, 18.94±0.2, 19.72±0.2, 22.15±0.2, 24.35±0.2, 25.12±0.2, 26.21±0.2, and 26.80±0.2.

In one embodiment, the X-ray powder diffraction pattern of the crystal form A further contains peaks at 2 or more than 2 of diffraction angles 2θ(°) values selected from the following group A-2:6.57±0.2, 8.71±0.2, 12.24±0.2, 14.07±0.2, 14.47±0.2, 15.48±0.2, 16.66±0.2, 17.70±0.2, 18.61±0.2, 20.24±0.2, 20.62±0.2, 22.72±0.2, and 27.46±0.2.

In one embodiment, the X-ray powder diffraction pattern of the crystal form A has peaks at 6 or more or all (such as 6, 7, 8, 9, 10, 11, 12, 13, 14, 15, etc.) of 2θ (°) values selected from the groups A-1 and A-2.

In one embodiment, the X-ray powder diffraction pattern of the crystal form A has peaks at diffraction angles 2θ(°) values of the following group: 8.71±0.2, 12.24±0.2, 14.07±0.2, 14.47±0.2, 14.75±0.2, 15.48±0.2, 15.97±0.2, 16.66±0.2, 17.20±0.2, 17.70±0.2, 18.61±0.2, 18.94±0.2, 19.72±0.2, 20.24±0.2, 20.62±0.2, 22.15±0.2, 22.72±0.2, 24.35±0.2, 25.12±0.2, 26.21±0.2, 26.80±0.2, 27.46±0.2.

In one embodiment, the X-ray powder diffraction pattern of the crystal form A has peaks at 2θ(°) values shown in table A1, and the relative intensity of each peak is as shown in table A1:

**Table A1**

| 2θ(°) | I/I₀ | 2θ(°) | I/I₀ | 2θ(°) | I/I₀ |
|---|---|---|---|---|---|
| 6.57 | W | 8.71 | M | 12.24 | M |
| 14.07 | M | 14.47 | M | 14.75 | S |
| 15.48 | M | 15.97 | VS | 16.66 | M |
| 17.20 | S | 17.70 | M | 18.61 | M |
| 18.94 | S | 19.72 | S | 20.24 | M |
| 20.62 | M | 22.15 | VS | 22.72 | M |
| 24.35 | S | 25.12 | S | 26.21 | VS |
| 26.80 | VS | 27.46 | M | | |

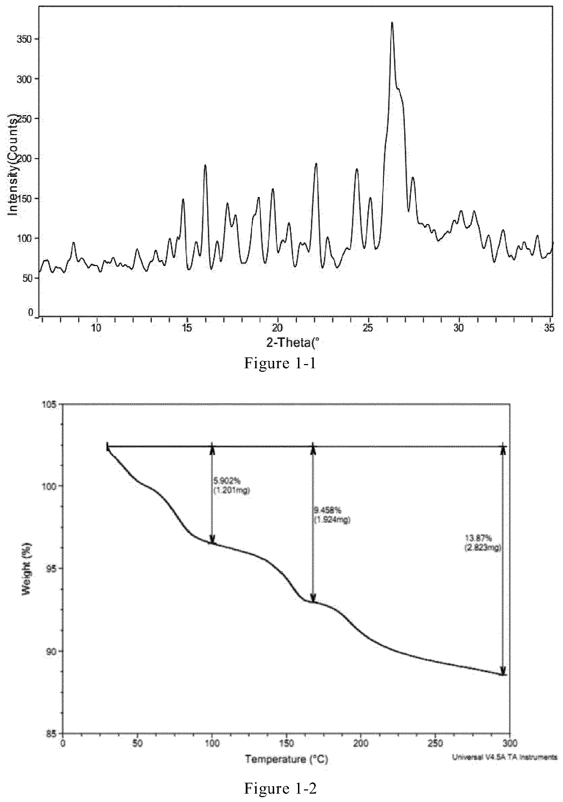

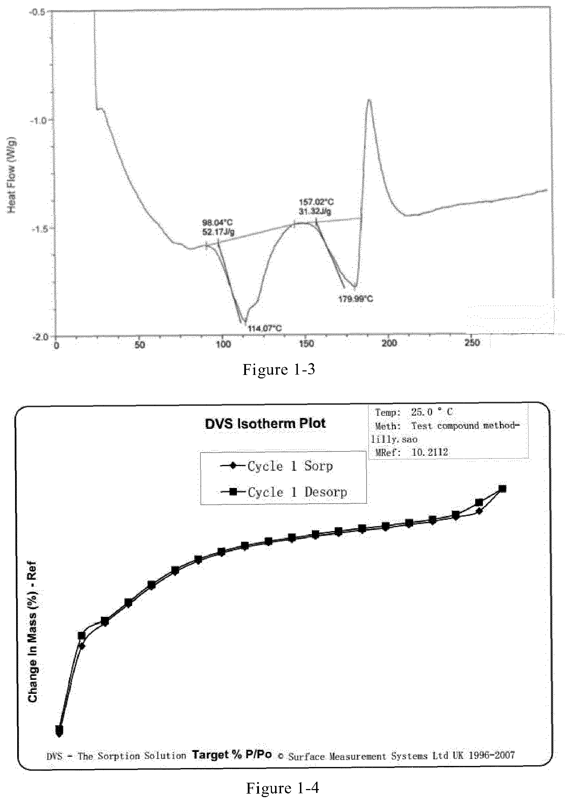

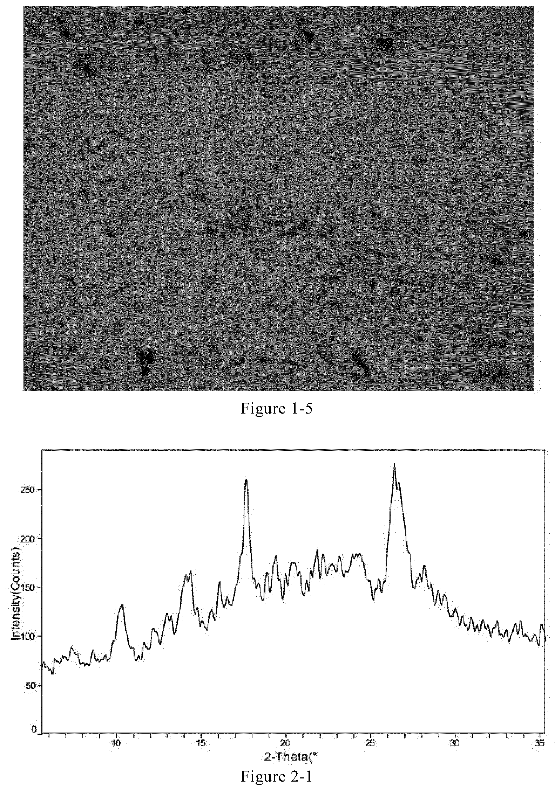

In one embodiment, the X-ray powder diffraction pattern of crystal form A is substantially as characterized in Figure 1-1.

In one embodiment, the crystal form A has a differential scanning calorimetry (DSC) spectrum basically the same as that shown in Figures 1-3.

In one embodiment, the crystal form A has a thermogravimetric analysis (TGA) spectrum basically the same as that shown in Figures 1-2.

In one embodiment, the polymorph is B-1 crystalline form of the sulfate of compound of formula X, i.e. crystal form B-1, the X-ray powder diffraction pattern of which has peaks at diffraction angles 2θ(°) values of the following group B-1-1: 10.27±0.2, 14.06±0.2, 14.41±0.2, 17.59±0.2, 19.39±0.2, 21.84±0.2, 26.38±0.2, and 26.68±0.2.

In one embodiment, the X-ray powder diffraction pattern of the crystal form B-1 has peaks at 2θ(°) values shown in table B1, and the relative intensity of each peak is as shown in table B1:

**Table B1**

| 2θ(°) | I/I₀ | 2θ(°) | I/I₀ |
|---|---|---|---|
| 10.27 | VS | 19.39 | S |
| 14.06 | VS | 21.84 | S |
| 14.41 | VS | 26.38 | VS |
| 17.59 | VS | 26.68 | VS |

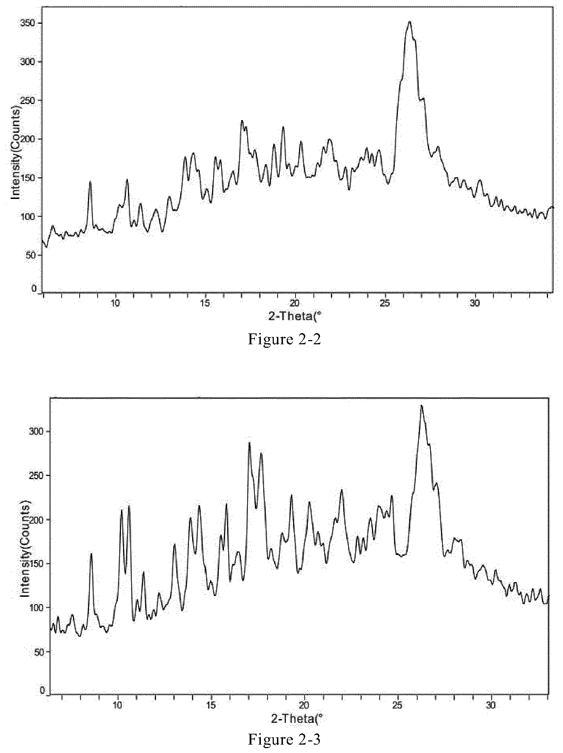

In one embodiment, the X-ray powder diffraction pattern of crystal form B-1 is substantially as characterized in Figure 2-1.

In one embodiment, the polymorph is B-2 crystalline form of the sulfate of compound of formula X, i.e. crystal form B-2, the X-ray powder diffraction pattern of which has peaks at diffraction angles 2θ(°) values of the following group B-2-1: 8.59±0.2, 10.64±0.2, 13.90±0.2, 14.38±0.2, 15.53±0.2, 17.05±0.2, 17.26±0.2, 17.75±0.2, 19.28±0.2, 21.85±0.2, 25.82±0.2, 26.32±0.2, and 26.62±0.2.

In one embodiment, the X-ray powder diffraction pattern of the crystal form B-2 further contains peaks at 2 or more than 2 of diffraction angles 2θ(°) values selected from the following group B-2-2: 11.39±0.2, 12.28±0.2, 12.97±0.2, 15.81±0.2, 18.79±0.2, and 20.31±0.2.

In one embodiment, the X-ray powder diffraction pattern of the crystal form B-2 has peaks at 6 or more or all (such as 6, 7, 8, 9, 10, 11, 12, 13, 14, 15, etc.) of 20 (°) values selected from the groups B-2-1 and B-2-2.

In one embodiment, the X-ray powder diffraction pattern of the crystal form B-2 has peaks at 2θ(°) values shown in table B2, and the relative intensity of each peak is as shown in table B2:

**Table B2**

| 2θ(°) | I/I₀ | 2θ(°) | I/I₀ | 2θ(°) | I/I₀ |
|---|---|---|---|---|---|
| 8.59 | VS | 15.53 | S | 19.28 | S |
| 10.64 | S | 15.81 | M | 20.31 | M |
| 11.39 | M | 17.05 | VS | 21.85 | S |
| 12.28 | M | 17.26 | S | 25.82 | VS |
| 12.97 | M | 17.75 | S | 26.32 | VS |
| 13.90 | S | 18.79 | M | 26.62 | VS |
| 14.38 | S | | | | |

In one embodiment, the X-ray powder diffraction pattern of crystal form B-2 is substantially as characterized in Figure 2-2.

In one embodiment, the polymorph is B-3 crystalline form of the sulfate of compound of formula X, i.e. crystal form B-3, the X-ray powder diffraction pattern of which has peaks at diffraction angles 2θ(°) values of the following group B-3-1: 8.59±0.2, 10.21±0.2, 10.60±0.2, 11.39±0.2, 13.03±0.2, 13.93±0.2, 14.38±0.2, 15.49±0.2, 15.82±0.2, 17.03±0.2, 17.71±0.2, 19.30±0.2, 20.23±0.2, 21.59±0.2, 21.97±0.2, 23.95±0.2, 24.62±0.2, 26.23±0.2, and 26.65±0.2.

In one embodiment, the X-ray powder diffraction pattern of the crystal form B-3 further contains peaks at diffraction angles 2θ(°) values selected from the following group B-3-2: 18.78±0.2.

In one embodiment, the X-ray powder diffraction pattern of the crystal form B-3 has peaks at 2θ(°) values shown in table B3, and the relative intensity of each peak is as shown in table B3:

**Table B3**

| 2θ(°) | I/I0 | 2θ(°) | I/I0 | 2θ(°) | I/I0 |
|---|---|---|---|---|---|
| 8.59 | VS | 15.49 | S | 21.59 | S |
| 10.21 | VS | 15.82 | VS | 21.97 | S |
| 10.60 | VS | 17.03 | VS | 23.95 | S |
| 11.39 | S | 17.71 | VS | 24.62 | S |
| 13.03 | S | 18.78 | M | 26.23 | VS |
| 13.93 | VS | 19.30 | VS | 26.65 | VS |
| 14.39 | VS | 20.23 | S | | |

In one embodiment, the X-ray powder diffraction pattern of crystal form B-3 is substantially as characterized in Figure 2-3.

In one embodiment, the polymorph is C crystalline form of the hydrobromide of compound of formula X, i.e. crystal form C, the X-ray powder diffraction pattern of which has peaks at diffraction angles 2θ(°) values of the following group C-1: 15.26±0.2, 15.91±0.2, 17.09±0.2, 18.43±0.2, 18.76±0.2, 19.49±0.2, 20.47±0.2, 21.91±0.2, 24.10±0.2, 24.88±0.2, 25.87±0.2, and 26.48±0.2.

In one embodiment, the X-ray powder diffraction pattern of the crystal form C further contains peaks at 2 or more than 2 of diffraction angles 2θ(°) values selected from the following group C-2: 8.69±0.2, 9.16±0.2, 10.82±0.2, 11.50±0.2, 14.62±0.2, 16.55±0.2, 17.50±0.2, 20.05±0.2, 21.33±0.2, and 22.62±0.2.

In one embodiment, the X-ray powder diffraction pattern of the crystal form C has peaks at 6 or more or all (such as 6, 7, 8, 9, 10, 11, 12, 13, 14, 15, etc.) of 2θ (°) values selected from the groups C-1 and C-2.

In one embodiment, the X-ray powder diffraction pattern of the crystal form C has peaks at diffraction angles 2θ(°) values of the following group: 8.69±0.2, 10.82±0.2, 11.50±0.2, 14.62±0.2, 15.26±0.2, 15.91±0.2, 17.09±0.2, 17.50±0.2, 18.43±0.2, 18.76±0.2, 19.49±0.2, 20.05±0.2, 20.47±0.2, 21.33±0.2, 21.91±0.2, 22.62±0.2, 24.10±0.2, 24.88±0.2, 25.87±0.2 and 26.48±0.2.

In one embodiment, the X-ray powder diffraction pattern of the crystal form C has peaks at 2θ(°) values shown in table C1, and the relative intensity of each peak is as shown in table C1:

**Table C1**

| 2θ(°) | I/I₀ | 2θ(°) | I/I₀ | 2θ(°) | I/I₀ |
|---|---|---|---|---|---|
| 8.69 | M | 17.09 | S | 21.33 | M |
| 9.16 | W | 17.50 | M | 21.91 | S |
| 10.82 | M | 18.43 | S | 22.62 | M |
| 11.50 | M | 18.76 | S | 24.10 | S |
| 14.62 | M | 19.49 | S | 24.88 | S |
| 15.26 | S | 20.05 | M | 25.87 | VS |
| 15.91 | S | 20.47 | S | 26.48 | VS |
| 16.55 | W | | | | |

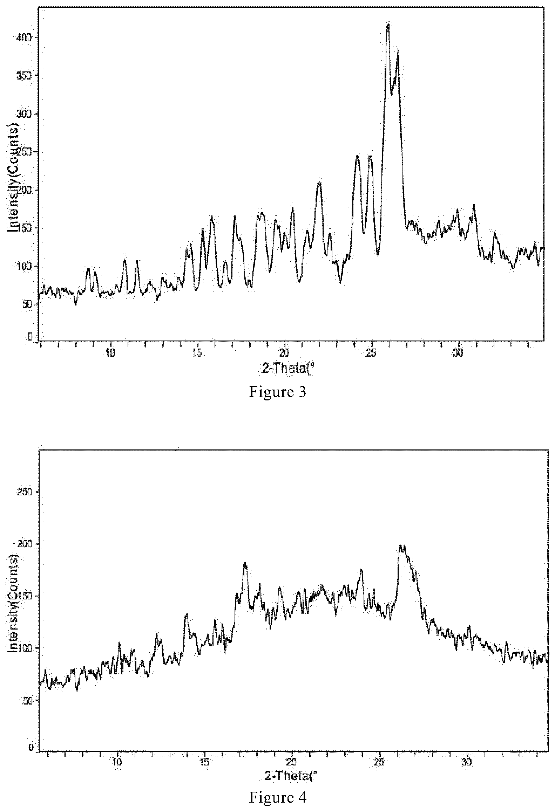

In one embodiment, the X-ray powder diffraction pattern of crystal form C is substantially as characterized in Figure 3.

In one embodiment, the polymorph is D crystalline form of the phosphate of compound of formula X, i.e. crystal form D, the X-ray powder diffraction pattern of which has peaks at diffraction angles 2θ(°) values of the following group D-1: 12.24±0.2, 13.93±0.2, 17.24±0.2, 18.18±0.2, 23.93±0.2, 26.38±0.2, and 26.68±0.2.

In one embodiment, the X-ray powder diffraction pattern of the crystal form D has peaks at 2θ(°) values shown in table D1, and the relative intensity of each peak is as shown in table D1:

**Table D1**

| 2θ(°) | I/I₀ | 2θ(°) | I/I₀ |
|---|---|---|---|
| 12.24 | VS | 23.93 | VS |
| 13.93 | VS | 26.38 | VS |
| 17.24 | VS | 26.68 | VS |
| 18.18 | VS | | |

In one embodiment, the X-ray powder diffraction pattern of crystal form D is substantially as characterized in Figure 4.

In one embodiment, the polymorph is E-1 crystalline form of the methanesulfonate of compound of formula X, i.e. crystal form E-1, the X-ray powder diffraction pattern of which has peaks at diffraction angles 2θ(°) values of the following group E-1-1: 8.56±0.2, 11.39±0.2, 17.47±0.2, 17.80±0.2, and 26.32±0.2.

In one embodiment, the X-ray powder diffraction pattern of the crystal form E-1 further contains peaks at 2 or more than 2 of diffraction angles 2θ(°) values selected from the following group E-1-2: 6.61±0.2, 12.79±0.2, 14.91±0.2, 16.81±0.2, 19.42±0.2, 20.23±0.2, 21.16±0.2, 21.40±0.2, 23.14±0.2, and 25.96±0.2.

In one embodiment, the X-ray powder diffraction pattern of the crystal form E-1 further contains peaks at 2 or more than 2 of diffraction angles 2θ(°) values selected from the following group E-1-3: 14.48±0.2, 15.79±0.2, 18.61±0.2, 19.96±0.2, 22.27±0.2, 24.07±0.2, 24.46±0.2, 25.75±0.2, and 27.67±0.2.

In one embodiment, the X-ray powder diffraction pattern of the crystal form E-1 has peaks at 6 or more or all (such as 6, 7, 8, 9, 10, 11, 12, 13, 14, 15, etc.) of 2θ (°) values selected from the groups E-1-1, E-1-2 and E-1-3.

In one embodiment, the X-ray powder diffraction pattern of the crystal form E-1 has peaks at diffraction angles 2θ(°) values of the following group: 6.61±0.2, 8.56±0.2, 11.39±0.2, 12.79±0.2, 14.91±0.2, 16.81±0.2, 17.47±0.2, 17.80±0.2, 19.42±0.2, 20.23±0.2, 21.16±0.2, 21.40±0.2, 23.14±0.2, 25.96±0.2, and 26.32±0.2.

In one embodiment, the X-ray powder diffraction pattern of the crystal form E-1 has peaks at 2θ(°) values shown in table E1, and the relative intensity of each peak is as shown in table E1:

**Table E1**

| 2θ(°) | I/I₀ | 2θ(°) | I/I₀ | 2θ(°) | I/I₀ |
|---|---|---|---|---|---|
| 6.61 | M | 17.47 | S | 22.27 | W |
| 8.56 | VS | 17.80 | VS | 23.14 | M |
| 11.39 | S | 18.61 | W | 24.07 | W |
| 12.79 | M | 19.42 | M | 24.46 | W |
| 14.48 | W | 19.96 | W | 25.75 | W |
| 14.91 | M | 20.23 | M | 25.96 | M |
| 15.79 | W | 21.16 | M | 26.32 | S |
| 16.81 | M | 21.40 | M | 27.67 | W |

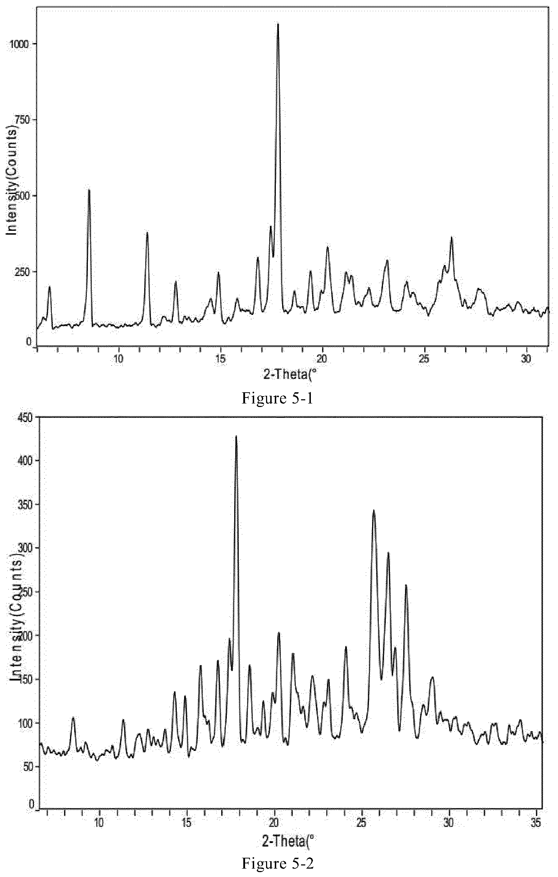

In one embodiment, the X-ray powder diffraction pattern of crystal form E-1 is substantially as characterized in Figure 5-1.

In one embodiment, the polymorph is E-2 crystalline form of the methanesulfonate of compound of formula X, i.e. crystal form E-2, the X-ray powder diffraction pattern of which has peaks at diffraction angles 2θ(°) values of the following group E-2-1: 15.79±0.2, 16.76±0.2, 17.41±0.2, 17.80±0.2, 20.26±0.2, 21.05±0.2, 24.10±0.2, 25.63±0.2, 26.53±0.2, 26.92±0.2, and 27.50±0.2.

In one embodiment, the X-ray powder diffraction pattern of the crystal form E-2 further contains peaks at 2 or more than 2 of diffraction angles 2θ(°) values selected from the following group E-2-2: 8.45±0.2, 11.33±0.2, 14.30±0.2, 14.89±0.2, 18.60±0.2, 19.36±0.2, 19.87±0.2, 22.16±0.2, 23.09±0.2, and 29.08±0.2.

In one embodiment, the X-ray powder diffraction pattern of the crystal form E-2 further contains peaks at 2 or more than 2 of diffraction angles 2θ(°) values selected from the following group E-2-3: 12.30±0.2, 12.75±0.2, 13.09±0.2, 13.29±0.2, 13.73±0.2, 16.03±0.2, 16.24±0.2, 22.77±0.2, and 28.49±0.2.

In one embodiment, the X-ray powder diffraction pattern of the crystal form E-2 has peaks at 6 or more or all (such as 6, 7, 8, 9, 10, 11, 12, 13, 14, 15, etc.) of 2θ (°) values selected from the groups E-2-1, E-2-2 and E-2-3.

In one embodiment, the X-ray powder diffraction pattern of the crystal form E-2 has peaks at diffraction angles 2θ(°) values of the following group: 8.45±0.2, 11.33±0.2, 14.30±0.2, 14.89±0.2, 15.79±0.2, 16.76±0.2, 17.41±0.2, 17.80±0.2, 18.60±0.2, 19.36±0.2, 19.87±0.2, 20.26±0.2, 21.05±0.2, 22.16±0.2, 23.09±0.2, 24.10±0.2, 25.63±0.2, 26.53±0.2, 26.92±0.2, 27.50±0.2, and 29.08±0.2.

In one embodiment, the X-ray powder diffraction pattern of the crystal form E-2 has peaks at 2θ(°) values shown in table E2, and the relative intensity of each peak is as shown in table E2:

**Table E2**

| 2θ(°) | I/I₀ | 2θ(°) | I/I₀ | 2θ(°) | I/I₀ |
|---|---|---|---|---|---|
| 8.45 | M | 16.03 | W | 22.16 | M |
| 11.33 | M | 16.24 | W | 22.77 | W |
| 12.30 | W | 16.76 | S | 23.09 | M |
| 12.75 | W | 17.41 | S | 24.10 | S |
| 13.09 | W | 17.80 | VS | 25.63 | VS |
| 13.29 | W | 18.60 | M | 26.53 | S |
| 13.73 | W | 19.36 | M | 26.92 | S |
| 14.30 | M | 19.87 | M | 27.50 | S |
| 14.89 | M | 20.26 | S | 28.49 | W |
| 15.79 | S | 21.05 | S | 29.08 | M |

In one embodiment, the X-ray powder diffraction pattern of crystal form E-2 is substantially as characterized in Figure 5-2.

In one embodiment, the polymorph is F crystalline form of the tartrate of compound of formula X, i.e. crystal form F, the X-ray powder diffraction pattern of which has peaks at diffraction angles 2θ(°) values of the following group F-1: 18.58±0.2, 19.84±0.2, 20.56±0.2, 24.88±0.2, 28.73±0.2, 29.45±0.2, 31.81±0.2, and 33.28±0.2.

In one embodiment, the X-ray powder diffraction pattern of the crystal form F has peaks at 2θ(°) values shown in table F1, and the relative intensity of each peak is as shown in table F1:

**Table F1**

| 2θ(°) | I/I0 | 2θ(°) | I/I0 |
|---|---|---|---|
| 18.58 | VS | 28.73 | M |
| 19.84 | M | 29.45 | M |
| 20.56 | VS | 31.81 | M |
| 24.88 | S | 33.28 | M |

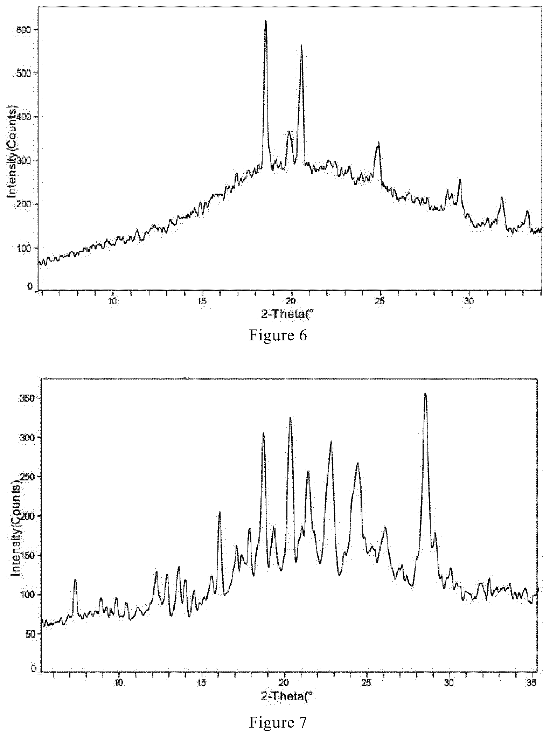

In one embodiment, the X-ray powder diffraction pattern of crystal form F is substantially as characterized in Figure 6.

In one embodiment, the polymorph is G crystalline form of the fumarate of compound of formula X, i.e. crystal form G, the X-ray powder diffraction pattern of which has peaks at diffraction angles 2θ(°) values of the following group G-1: 16.06±0.2, 18.76±0.2, 20.32±0.2, 21.49±0.2, 22.52±0.2, 22.84±0.2, 24.32±0.2, 24.50±0.2, 26.06±0.2, and 28.48±0.2.

In one embodiment, the X-ray powder diffraction pattern of the crystal form G further contains peaks at 2 or more than 2 of diffraction angles 2θ(°) values selected from the following group G-2: 7.35±0.2, 12.25±0.2, 12.88±0.2, 13.60±0.2, 13.96±0.2, 15.50±0.2, 17.03±0.2, 17.80±0.2, 19.34±0.2, and 20.93±0.2.

In one embodiment, the X-ray powder diffraction pattern of the crystal form G has peaks at 6 or more or all (such as 6, 7, 8, 9, 10, 11, 12, 13, 14, 15, etc.) of 2θ (°) values selected from the groups G-1 and G-2.

In one embodiment, the X-ray powder diffraction pattern of the crystal form G has peaks at 2θ(°) values shown in table G1, and the relative intensity of each peak is as shown in table G1:

**Table G1**

| 2θ(°) | I/I₀ | 2θ(°) | I/I₀ |
|---|---|---|---|
| 7.35 | M | 19.34 | M |
| 12.25 | M | 20.32 | VS |
| 12.88 | M | 20.93 | M |
| 13.60 | M | 21.49 | VS |
| 13.96 | M | 22.52 | S |
| 15.50 | M | 22.84 | VS |
| 16.06 | S | 24.32 | S |
| 17.03 | M | 24.50 | S |
| 17.80 | M | 26.06 | S |
| 18.76 | VS | 28.48 | VS |

In one embodiment, the X-ray powder diffraction pattern of crystal form G is substantially as characterized in Figure 7.

In one embodiment, the polymorph is H-1 crystalline form of the succinate of compound of formula X, i.e. crystal form H-1, the X-ray powder diffraction pattern of which has peaks at diffraction angles 2θ(°) values of the following group H-1-1: 21.70±0.2.

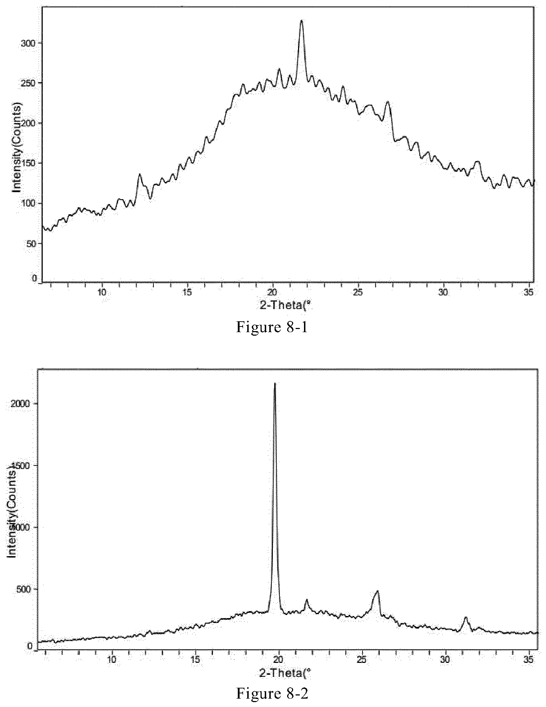

In one embodiment, the polymorph is H-1 crystalline form of the succinate of compound of formula X, i.e. crystal form H-1, the X-ray powder diffraction pattern of which is substantially as characterized in Figure 8-1.

In one embodiment, the polymorph is H-2 crystalline form of the succinate of compound of formula X, i.e. crystal form H-2, the X-ray powder diffraction pattern of which has peaks at diffraction angles 2θ(°) values of the following group H-2-1: 19.78±0.2, 21.63±0.2, 25.96±0.2, and 31.23±0.2.

In one embodiment, the polymorph is H-2 crystalline form of the succinate of compound of formula X, i.e. crystal form H-2, the X-ray powder diffraction pattern of which is substantially as characterized in Figure 8-2.

In one embodiment, the polymorph is H-3 crystalline form of the succinate of compound of formula X, i.e. crystal form H-3, the X-ray powder diffraction pattern of which has peaks at diffraction angles 2θ(°) values of the following group H-3-1: 12.20±0.2, 19.72±0.2, 19.84±0.2, 25.82±0.2, and 31.21±0.2.

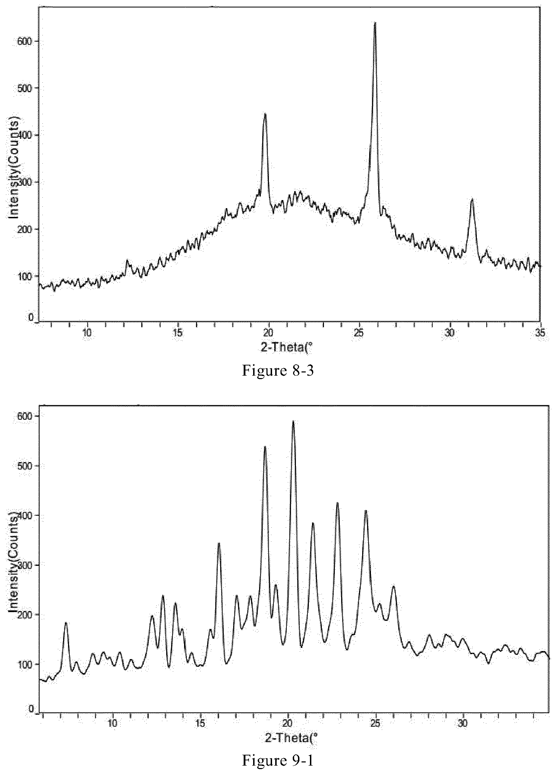

In one embodiment, the polymorph is H-3 crystalline form of the succinate of compound of formula X, i.e. crystal form H-3, the X-ray powder diffraction pattern of which is substantially as characterized in Figure 8-3.

In one embodiment, the polymorph is crystal form I of the compound of formula X, the X-ray powder diffraction pattern of which has peaks at diffraction angles 2θ(°) values of the following group I-1: 16.01±0.2, 18.64±0.2, 20.27±0.2, 21.40±0.2, 22.84±0.2, and 24.49±0.2.

In one embodiment, the X-ray powder diffraction pattern of the crystal form I further contains peaks at 2 or more than 2 of diffraction angles 2θ(°) values selected from the following group I-2: 7.28±0.2, 12.23±0.2, 12.88±0.2, 13.55±0.2, 17.05±0.2, 17.83±0.2, 19.36±0.2, and 26.06±0.2.

In one embodiment, the X-ray powder diffraction pattern of the crystal form I further contains peaks at 2 or more than 2 of diffraction angles 2θ(°) values selected from the following group I-3: 8.83±0.2, 9.48±0.2, 10.39±0.2, 13.94±0.2, 15.58±0.2, 25.19±0.2, and 28.02±0.2.

In one embodiment, the X-ray powder diffraction pattern of the crystal form I has peaks at 6 or more or all (such as 6, 7, 8, 9, 10, 11, 12, 13, 14, 15, etc.) of 29 (°) values selected from the groups I-1, I-2 and I-3.

In one embodiment, the X-ray powder diffraction pattern of the crystal form I has peaks at diffraction angles 2θ(°) values of the following group: 7.28±0.2, 12.23±0.2, 12.88±0.2, 13.55±0.2, 16.01±0.2, 17.05±0.2, 17.83±0.2, 18.64±0.2, 19.36±0.2, 20.27±0.2, 21.40±0.2, 22.84±0.2, 24.49±0.2, and 26.06±0.2.

In one embodiment, the X-ray powder diffraction pattern of the crystal form I has peaks at diffraction angles 2θ(°) values of the following group: 7.28±0.2, 9.48±0.2, 12.23±0.2, 12.88±0.2, 13.55±0.2, 13.94±0.2, 15.58±0.2, 16.01±0.2, 17.05±0.2, 17.83±0.2, 18.64±0.2, 19.36±0.2, 20.27±0.2, 21.40±0.2, 22.84±0.2, 24.49±0.2, 26.06±0.2, and 28.02±0.2.

In one embodiment, the X-ray powder diffraction pattern of the crystal form I has peaks at 2θ(°) values shown in table II, and the relative intensity of each peak is as shown in table I1:

**Table I1**

| 2θ(°) | I/I₀ | 2θ(°) | I/I₀ | 2θ(°) | I/I₀ |
|---|---|---|---|---|---|
| 7.28 | S | 13.94 | M | 20.27 | VS |
| 8.83 | W | 15.58 | M | 21.40 | VS |
| 9.48 | M | 16.01 | VS | 22.84 | VS |
| 10.39 | W | 17.05 | S | 24.49 | VS |
| 12.23 | S | 17.83 | S | 25.19 | W |
| 12.88 | S | 18.64 | VS | 26.06 | S |
| 13.55 | S | 19.36 | S | 28.02 | M |

In one embodiment, the X-ray powder diffraction pattern of crystal form I is substantially as characterized in Figure 9-1.

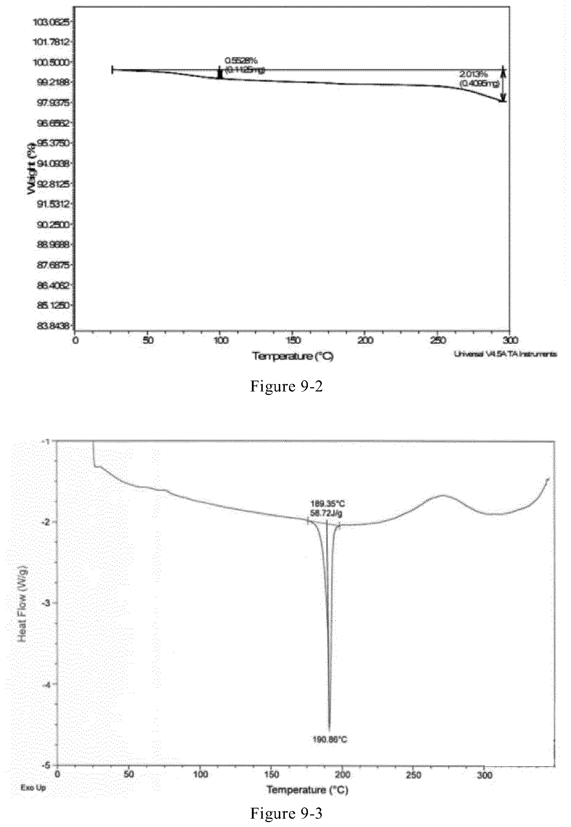

In one embodiment, the crystal form I has a differential scanning calorimetry (DSC) spectrum basically the same as that shown in Figures 9-3.

In one embodiment, the crystal form I has a thermogravimetric analysis (TGA) spectrum basically the same as that shown in Figures 9-2.

In one embodiment, the polymorph is crystal form II of the compound of formula X, the X-ray powder diffraction pattern of which has peaks at diffraction angles 2θ(°) values of the group II-1: 7.32±0.2, 9.84±0.2, 13.56±0.2, 17.47±0.2, 22.73±0.2, 24.37±0.2, and 25.09±0.2.

In one embodiment, the X-ray powder diffraction pattern of the crystal form II further contains peaks at 2 or more than 2 of diffraction angles 2θ(°) values selected from the following group II-2: 12.37±0.2, 12.82±0.2, 13.89±0.2, 15.53±0.2, 15.98±0.2, 17.03±0.2, 17.82±0.2, 18.64±0.2, 19.20±0.2, 19.84±0.2, 20.20±0.2, 20.30±0.2, 21.36±0.2, 24.01±0.2, and 29.84±0.2.

In one embodiment, the X-ray powder diffraction pattern of the crystal form II has peaks at 6 or more or all (such as 6, 7, 8, 9, 10, 11, 12, 13, 14, 15, etc.) of 20 (°) values selected from the groups II-1 and II-2.

In one embodiment, the X-ray powder diffraction pattern of the crystal form II has peaks at diffraction angles 2θ(°) values of the following group: 7.32±0.2, 9.84±0.2, 12.37±0.2, 13.56±0.2, 15.53±0.2, 15.98±0.2, 17.03±0.2, 17.47±0.2, 17.82±0.2, 18.64±0.2, 20.20±0.2, 20.30±0.2, 21.36±0.2, 22.73±0.2, 24.37±0.2, 25.09±0.2, and 29.84±0.2.

In one embodiment, the X-ray powder diffraction pattern of the crystal form II has peaks at 2θ(°) values shown in table III, and the relative intensity of each peak is as shown in table III:

**Table III**

| 2θ(°) | I/I₀ | 2θ(°) | I/I₀ | 2θ(°) | I/I₀ |
|---|---|---|---|---|---|
| 7.32 | VS | 17.03 | M | 20.30 | M |
| 9.84 | S | 17.47 | S | 21.36 | M |
| 12.37 | M | 17.82 | M | 22.73 | S |
| 12.82 | W | 18.64 | M | 24.01 | W |
| 13.56 | S | 19.20 | W | 24.37 | S |
| 13.89 | W | 19.84 | W | 25.09 | S |
| 15.53 | M | 20.20 | M | 29.84 | M |
| 15.98 | M | | | | |

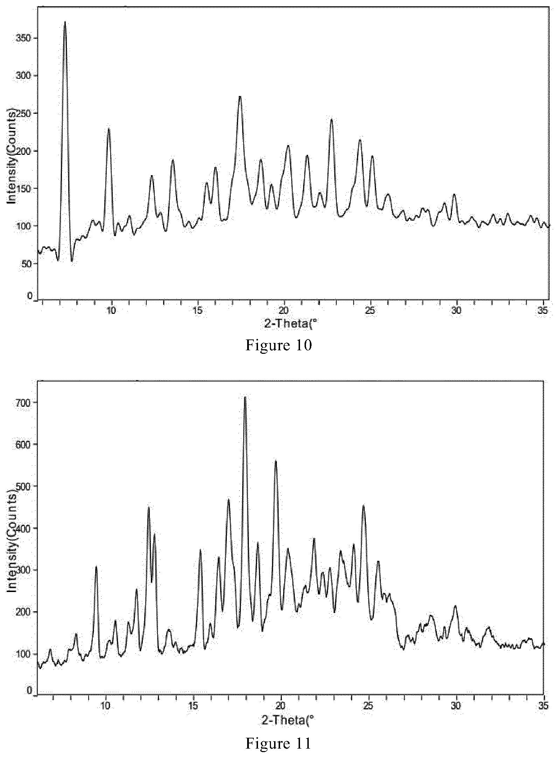

In one embodiment, the X-ray powder diffraction pattern of crystal form II is substantially as characterized in Figure 10.

In one embodiment, the polymorph is crystal form III of the compound of formula X, the X-ray powder diffraction pattern of which has peaks at diffraction angles 2θ(°) values of the group III-1: 9.52±0.2, 11.77±0.2, 12.43±0.2, 12.78±0.2, 15.31±0.2, 16.33±0.2, 16.84±0.2, 17.83±0.2, 18.49±0.2, 19.57±0.2, 20.15±0.2, 21.71±0.2, 23.26±0.2, 23.84±0.2, 24.52±0.2, and 25.30±0.2.

In one embodiment, the X-ray powder diffraction pattern of the crystal form III further contains peaks at 2 or more than 2 of diffraction angles 2θ(°) values selected from the following group III-2: 7.00±0.2, 8.35±0.2, 11.35±0.2, 13.65±0.2, 17.20±0.2, 22.18±0.2, 22.60±0.2, 25.70±0.2, 28.33±0.2, and 29.77±0.2.

In one embodiment, the X-ray powder diffraction pattern of the crystal form III has peaks at 6 or more or all (such as 6, 7, 8, 9, 10, 11, 12, 13, 14, 15, etc.) of 2θ (°) values selected from the groups III-1 and III-2.

In one embodiment, the X-ray powder diffraction pattern of the crystal form III has peaks at diffraction angles 2θ(°) values of the following group: 8.35±0.2, 9.52±0.2, 11.35±0.2, 11.77±0.2, 12.43±0.2, 12.78±0.2, 13.65±0.2, 15.31±0.2, 16.33±0.2, 16.84±0.2, 17.20±0.2, 17.83±0.2, 18.49±0.2, 19.57±0.2, 20.15±0.2, 21.71±0.2, 22.18±0.2, 22.60±0.2, 23.26±0.2, 23.84±0.2, 24.52±0.2, 25.30±0.2, 25.70±0.2, 28.33±0.2, and 29.77±0.2.

In one embodiment, the X-ray powder diffraction pattern of the crystal form III has peaks at 2θ(°) values shown in table III1, and the relative intensity of each peak is as shown in table III1:

**Table III1**

| 2θ(°) | I/I₀ | 2θ(°) | I/I₀ | 2θ(°) | I/I₀ |
|---|---|---|---|---|---|
| 7.00 | W | 16.33 | S | 22.60 | M |
| 8.35 | M | 16.84 | S | 23.26 | S |
| 9.52 | S | 17.20 | M | 23.84 | S |
| 11.35 | M | 17.83 | VS | 24.52 | VS |
| 11.77 | S | 18.49 | S | 25.30 | S |
| 12.43 | VS | 19.57 | VS | 25.70 | M |
| 12.78 | S | 20.15 | S | 28.33 | M |
| 13.65 | M | 21.71 | S | 29.77 | M |
| 15.31 | S | 22.18 | M | | |

In one embodiment, the X-ray powder diffraction pattern of crystal form III is substantially as characterized in Figure 11.

In a second aspect of the disclosure, there is provided a process for preparing the pharmaceutically acceptable salt of the compound of formula X, the polymorph of the compound of formula X or the polymorph of pharmaceutically acceptable salt of the compound of formula X according to the first aspect of the present disclosure,
wherein the process for preparing the pharmaceutically acceptable salt of the compound of formula X or the polymorph of pharmaceutically acceptable salt of the compound of formula X comprises steps:
(1) conducting a salt forming reaction with the compound of formula X and an acid to form a pharmaceutically acceptable salt; and
(2) crystallizing the pharmaceutically acceptable salt of the compound of formula X formed in step (1) to obtain a polymorph.

In one embodiment, in step (1), the compound of formula X is reacted with an acid to form a salt in the presence of a solvent, the solvent is selected from the group consisting of water, acetonitrile, ethanol, isopropanol, acetone, ethyl acetate, methyl tert-butyl ether, tetrahydrofuran, n-heptane, and dimethyl sulfoxide. In another embodiment, in step (1), the compound of formula X is reacted with an acid to form a salt in the presence of a solvent, the solvent is isopropanol, acetone, ethyl acetate, or acetonitrile.

In one embodiment, in step (1), the acid is selected from a group consisting of hydrochloric acid, sulfuric acid, hydrobromic acid, phosphoric acid, methanesulfonic acid, L-tartaric acid, fumaric acid and succinic acid.

In one embodiment, in step (2), the crystallization process is suspension centrifugation, suspension stirring, slow volatilization, or cooling crystallization.

In one embodiment, the preparation method of the crystal form A comprises the following steps:
(A-1) dissolving or suspending the compound of formula X in a solvent; and
(A-2) adding hydrochloric acid to the mixture of step (A-1), heating and stirring under suspension, followed by cooling and separating to obtain the crystal form A.

In one embodiment, in step (A-1), the solvent is selected from a group consisting of ethyl acetate, acetone and acetonitrile.

In one embodiment, in step (A-2), the molar ratio of hydrochloric acid to the compound of formula X is (0.2∼2.5): 1. In another embodiment, in step (A-2), the molar ratio of hydrochloric acid to the compound of formula X is (1∼2): 1. In another embodiment, in step (A-2), the molar ratio of hydrochloric acid to the compound of formula X is 1.2:1.

In one embodiment, in step (A-2), the concentration of hydrochloric acid is 0.25M-1M, for example 1M.

In one embodiment, in step (A-2), the mixture is heated, stirred under suspension, and then cooled, and solids precipitate.

In one embodiment, in step (A-2), the heating temperature is 25°C-60°C, for example 40°C-60°C.

In one embodiment, in step (A-2), the time for heating is 0.5-12 hours, for example 3-8 hours.

In one embodiment, in step (A-2), cooling to 0°C-30°C, for example to room temperature, is performed.

In one embodiment, in step (A-2), the cooling time is 0.5-30 hours, for example 12-24 hours.

In one embodiment, in step (A-2), the separation is carried out by centrifugal separation or filtration.

In one embodiment, the preparation method of the crystal form B-1 comprises the following steps:
(B-1-1) suspending the compound of formula X in a solvent;
(B-1-2) adding sulfuric acid to the mixture of step (B-1-1), heating and stirring under suspension, followed by cooling and separating to obtain the crystal form B-1.

In one embodiment, in step (B-1-1), the solvent is isopropanol.

In one embodiment, in step (B-1-2), the molar ratio of sulfuric acid to the compound of formula X is (0.2∼2.5):1. In another embodiment, in step (B-1-2), the molar ratio of sulfuric acid to the compound of formula X is (1∼2):1. In another embodiment, in step (B-1-2), the molar ratio of sulfuric acid to the compound of formula X is 1.2:1.

In one embodiment, in step (B-1-2), the concentration of sulfuric acid is 0.25M-1M, for example 0.5M.

In one embodiment, in step (B-1-2), the heating temperature is 25°C-60°C, for example 40°C-60°C.

In one embodiment, in step (B-1-2), the time for heating is 0.5-12 hours, for example 3-8 hours.

In one embodiment, in step (B-1-2), cooling to 0°C-30°C, for example to room temperature, is performed.

In one embodiment, in step (B-1-2), the cooling time is 0.5-30 hours, for example 12-24 hours.

In one embodiment, in step (B-1-2), the separation is carried out by centrifugal separation or filtration.

In one embodiment, the preparation method of the crystal form B-2 comprises the following steps:
(B-2-1) suspending the compound of formula X in a solvent;
(B-2-2) adding sulfuric acid to the mixture of step (B-2-1), heating and stirring under suspension, followed by cooling and separating to obtain the crystal form B-2.

In one embodiment, in step (B-2-1), the solvent is ethyl acetate or acetonitrile.

In one embodiment, in step (B-2-2), the molar ratio of sulfuric acid to the compound of formula X is (0.2∼2.5): 1. In another embodiment, in step (B-2-2), the molar ratio of sulfuric acid to the compound of formula X is (1∼2); 1. In another embodiment, in step (B-2-2), the molar ratio of sulfuric acid to the compound of formula X is 1.2:1.

In one embodiment, in step (B-2-2), the concentration of sulfuric acid is 0.25M-1M, for example 0.5M.

In one embodiment, in step (B-2-2), the heating temperature is 25°C-60°C, for example 40°C-60°C.

In one embodiment, in step (B-2-2), the time for heating is 0.5-12 hours, for example 3-8 hours.

In one embodiment, in step (B-2-2), cooling to 0°C-30°C, for example to room temperature, is performed.

In one embodiment, in step (B-2-2), the cooling time is 0.5-30 hours, for example 12-24 hours.

In one embodiment, in step (B-2-2), the separation is carried out by centrifugal separation or filtration.

In one embodiment, the preparation method of the crystal form B-3 comprises the following steps:
(B-3-1) suspending the compound of formula X in a solvent;
(B-3-2) adding sulfuric acid to the mixture of step (B-3-1), heating and stirring under suspension, followed by cooling and separating to obtain the crystal form B-3.

In one embodiment, in step (B-3-1), the solvent is acetone.

In one embodiment, in step (B-3-2), the molar ratio of sulfuric acid to the compound of formula X is (0.2∼2.5):1. In another embodiment, in step (B-3-2), the molar ratio of sulfuric acid to the compound of formula X is (1∼2); 1. In another embodiment, in step (B-3-2), the molar ratio of sulfuric acid to the compound of formula X is 1.2:1.

In one embodiment, in step (B-3-2), the concentration of sulfuric acid is 0.25M-1M, for example 0.5M.

In one embodiment, in step (B-3-2), the heating temperature is 25°C-60°C, for example 40°C-60°C.

In one embodiment, in step (B-3-2), the time for heating is 0.5-12 hours, for example 3-8 hours.

In one embodiment, in step (B-3-2), cooling to 0°C-30°C, for example to room temperature, is performed.

In one embodiment, in step (B-3-2), the cooling time is 0.5-30 hours, for example 12-24 hours.

In one embodiment, in step (B-3-2), the separation is carried out by centrifugal separation or filtration.

In one embodiment, the preparation method of the crystal form C comprises the following steps:
(C-1) suspending the compound of formula X in a solvent;
(C-2) adding hydrobromic acid to the mixture of step (C-1), heating and stirring under suspension, followed by cooling and separating to obtain the crystal form C.

In one embodiment, in step (C-1), the solvent is ethyl acetate, acetone or acetonitrile.

In one embodiment, in step (C-2), the molar ratio of hydrobromic acid to the compound of formula X is (0.2∼2.5): 1. In another embodiment, in step (C-2), the molar ratio of hydrobromic acid to the compound of formula X is (1∼2): 1. In another embodiment, in step (C-2), the molar ratio of hydrobromic acid to the compound of formula X is 1.2:1.

In one embodiment, in step (C-2), the concentration of hydrobromic acid is 0.25M-1M, for example 1M.

In one embodiment, in step (C-2), the heating temperature is 25°C-60°C, for example 40°C-60°C.

In one embodiment, in step (C-2), the time for heating is 0.5-12 hours, for example 3-8 hours.

In one embodiment, in step (C-2), cool to to 0°C-30°C, for example to room temperature, is performed.

In one embodiment, in step (C-2), the cooling time is 0.5-30 hours, for example 12-24 hours.

In one embodiment, in step (C-2), the separation is carried out by centrifugal separation or filtration.

In one embodiment, the preparation method of the crystal form D comprises the following steps:
(D-1) suspending the compound of formula X in a solvent;
(D-2) adding phosphoric acid to the mixture of step (D-1), heating and stirring under suspension, followed by cooling and separating to obtain the crystal form D.

In one embodiment, in step (D-1), the solvent is ethyl acetate.

In one embodiment, in step (D-2), the molar ratio of phosphoric acid to the compound of formula X is (0.2∼2.5): 1. In another embodiment, in step (D-2), the molar ratio of phosphoric acid to the compound of formula X is (1∼2): 1. In another embodiment, in step (D-2), the molar ratio of phosphoric acid to the compound of formula X is 1.2:1.

In one embodiment, in step (D-2), the concentration of phosphoric acid is 0.25M-1M, for example 1M.

In one embodiment, in step (D-2), the heating temperature is 25°C-60°C, for example 40°C-60°C.

In one embodiment, in step (D-2), the time for heating is 0.5-12 hours, for example 3-8 hours.

In one embodiment, in step (D-2), cool to 0°C-30°C, for example room temperature.

In one embodiment, in step (D-2), the cooling time is 0.5-30 hours, for example 12-24 hours.

In one embodiment, in step (D-2), the separation is carried out by centrifugal separation or filtration.

In one embodiment, the preparation method of the crystal form E-1 comprises the following steps:
(E-1-1) suspending the compound of formula X in a solvent;
(E-1-2) adding methanesulfonic acid to the mixture of step (E-1-1), heating and stirring under suspension, followed by cooling and separating to obtain the crystal form E-1.

In one embodiment, in step (E-1-1), the solvent is isopropanol.

In one embodiment, in step (E-1-2), the molar ratio of methanesulfonic acid to the compound of formula X is (0.2∼2.5): 1. In another embodiment, in step (E-1-2), the molar ratio of methanesulfonic acid to the compound of formula X is (1∼2): 1. In another embodiment, in step (E-1-2), the molar ratio of methanesulfonic acid to the compound of formula X is 1.2:1.

In one embodiment, in step (E-1-2), the concentration of methanesulfonic acid is 0.25M-1M, for example 1M.

In one embodiment, in step (E-1-2), the heating temperature is 25°C-60°C, for example 40°C-60°C.

In one embodiment, in step (E-1-2), the time for heating is 0.5-12 hours, for example 3-8 hours.

In one embodiment, in step (E-1-2), cooling to 0°C-30°C, for example to room temperature, is performed.

In one embodiment, in step (E-1-2), the cooling time is 0.5-30 hours, for example 12-24 hours.

In one embodiment, in step (E-1-2), the separation is carried out by centrifugal separation or filtration.

In one embodiment, the preparation method of the crystal form E-2 comprises the following steps:
(E-2-1) suspending the compound of formula X in a solvent;
(E-2-2) adding the methanesulfonic acid to the mixture of step (E-2-1), heating and stirring under suspension, followed by cooling and separating to obtain the crystal form E-2.

In one embodiment, in step (E-2-1), the solvent is ethyl acetate or acetone.

In one embodiment, in step (E-2-2), the molar ratio of methanesulfonic acid to the compound of formula X is (0.2∼2.5): 1. In another embodiment, in step (E-2-2), the molar ratio of methanesulfonic acid to the compound of formula X is (1∼2): 1. In another embodiment, in step (E-2-2), the molar ratio of methanesulfonic acid to the compound of formula X is 1.2:1.

In one embodiment, in step (E-2-2), the concentration of methanesulfonic acid is 0.25M-1M, for example 1M.

In one embodiment, in step (E-2-2), the heating temperature is 25°C-60°C, for example 40°C-60°C.

In one embodiment, in step (E-2-2), the time for heating is 0.5-12 hours, for example 3-8 hours.

In one embodiment, in step (E-2-2), cooling to 0°C-30°C, for example to room temperature, is performed.

In one embodiment, in step (E-2-2), the cooling time is 0.5-30 hours, for example 12-24 hours.

In one embodiment, in step (E-2-2), the separation is carried out by centrifugal separation or filtration.

In one embodiment, the preparation method of the crystal form F comprises the following steps:
(F-1) suspending the compound of formula X in a solvent;
(F-2) adding tartaric acid to the mixture of step (F-1), heating and stirring under suspension, followed by cooling and separating to obtain the crystal form F.

In one embodiment, in step (F-1), the solvent is ethyl acetate.

In one embodiment, in step (F-2), the molar ratio of tartaric acid to the compound of formula X is (0.2∼2.5): 1. In another embodiment, in step (F-2), the molar ratio of tartaric acid to the compound of formula X is (1∼2); 1. In another embodiment, in step (F-2), the molar ratio of tartaric acid to the compound of formula X is 1.2:1.

In one embodiment, in step (F-2), the concentration of tartaric acid is 0.25M-1M, for example 1M.

In one embodiment, in step (F-2), the heating temperature is 25°C-60°C, for example 40°C-60°C.

In one embodiment, in step (F-2), the time for heating is 0.5-12 hours, for example 3-8 hours.

In one embodiment, in step (F-2), cooling to 0°C-30°C, for example to room temperature, is performed.

In one embodiment, in step (F-2), the cooling time is 0.5-30 hours, for example 12-24 hours.

In one embodiment, in step (F-2), the separation is carried out by centrifugal separation or filtration.

In one embodiment, the preparation method of the crystal form G comprises the following steps:
(G-1) dissolving or suspending the compound of formula X in a solvent;
(G-2) adding fumaric acid to the mixture of step (G-1), heating and stirring under suspension, followed by cooling and separating to obtain the crystal form G.

In one embodiment, in step (G-1), the solvent is isopropanol, acetonitrile, ethyl acetate, tetrahydrofuran or acetone.

In one embodiment, in step (G-2), after cooling, an anti-solvent or crystal seed is added.

In one embodiment, in step (G-2), the anti-solvent is methyl tert-butyl ether.

In one embodiment, in step (G-2), the molar ratio of fumaric acid to the compound of formula X is (0.2∼2.5): 1. In another embodiment, in step (G-2), the molar ratio of fumaric acid to the compound of formula X is (1∼2); 1. In another embodiment, in step (G-2), the molar ratio of fumaric acid to the compound of formula X is 1.2:1.

In one embodiment, in step (G-2), the concentration of fumaric acid is 0.25M-1M.

In one embodiment, in step (G-2), the heating temperature is 25°C-60°C, for example 40°C-60°C.

In one embodiment, in step (G-2), the time for heating is 0.5-12 hours, for example 3-8 hours.

In one embodiment, in step (G-2), cooling to 0°C-30°C, for to example room temperature, is performed.

In one embodiment, in step (G-2), the cooling time is 0.5-30 hours, for example 12-24 hours.

In one embodiment, in step (G-2), the separation is carried out by centrifugal separation or filtration.

In one embodiment, the preparation method of the crystal form H-1 comprises the following steps:
(H-1-1) suspending the compound of formula X in a solvent;
(H-1-2) adding succinic acid to the mixture of step (H-1-1), heating and stirring under suspension, followed by cooling and separating to obtain the crystal form H-1.

In one embodiment, in step (H-1-1), the solvent is isopropanol or acetonitrile.

In one embodiment, in step (H-1-2), the molar ratio of succinic acid to the compound of formula X is (0.2∼2.5):1. In another embodiment, in step (H-1-2), the molar ratio of succinic acid to the compound of formula X is (1∼2):1. In another embodiment, in step (H-1-2), the molar ratio of succinic acid to the compound of formula X is 1.2:1.

In one embodiment, in step (H-1-2), the concentration of succinic acid is 0.25M-1M, for example 0.5M.

In one embodiment, in step (H-1-2), the heating temperature is 25°C-60°C, for example 40°C-60°C.

In one embodiment, in step (H-1-2), the time for heating is 0.5-12 hours, for example 3-8 hours.

In one embodiment, in step (H-1-2), cooling to 0°C-30°C, for example to room temperature, is performed.

In one embodiment, in step (H-1-2), the cooling time is 0.5-30 hours, for example 12-24 hours.

In one embodiment, in step (H-1-2), the separation is carried out by centrifugal separation or filtration.

In one embodiment, the preparation method of the crystal form H-2 comprises the following steps:
(H-2-1) suspending the compound of formula X in a solvent;
(H-2-2) adding succinic acid to the mixture of step (H-2-1), heating and stirring under suspension, followed by cooling and separating to obtain the crystal form H-2.

In one embodiment, in step (H-2-1), the solvent is ethyl acetate.

In one embodiment, in step (H-2-2), the molar ratio of succinic acid to the compound of formula X is (0.2∼2.5):1. In another embodiment, in step (H-2-2), the molar ratio of succinic acid to the compound of formula X is (1∼2):1. In another embodiment, in step (H-2-2), the molar ratio of succinic acid to the compound of formula X is 1.2:1.

In one embodiment, in step (H-2-2), the concentration of succinic acid is 0.25M-1M, for example 0.5M.

In one embodiment, in step (H-2-2), the heating temperature is 25°C-60°C, for example 40°C-60°C.

In one embodiment, in step (H-2-2), the time for heating is 0.5-12 hours, for example 3-8 hours.

In one embodiment, in step (H-2-2), cooling to 0°C-30°C, for example to room temperature, is performed.

In one embodiment, in step (H-2-2), the cooling time is 0.5-30 hours, for example 12-24 hours.

In one embodiment, in step (H-2-2), the separation is carried out by centrifugal separation or filtration.

In one embodiment, the preparation method of the crystal form H-3 comprises the following steps:
(H-3-1) suspending the compound of formula X in a solvent;
(H-3-2) adding succinic acid to the mixture of step (H-3-1), heating and stirring under suspension, followed by cooling and separating to obtain the crystal form H-3.

In one embodiment, in step (H-3-1), the solvent is acetone.

In one embodiment, in step (H-3-2), the molar ratio of succinic acid to the compound of formula X is (0.2∼2.5):1. In another embodiment, in step (H-3-2), the molar ratio of succinic acid to the compound of formula X is (1∼2):1. In another embodiment, in step (H-3-2), the molar ratio of succinic acid to the compound of formula X is 1.2:1.

In one embodiment, in step (H-3-2), the concentration of succinic acid is 0.25M-1M, for example 0.5M.

In one embodiment, in step (H-3-2), the heating temperature is 25°C-60°C, for example 40°C-60°C.

In one embodiment, in step (H-3-2), the time for heating is 0.5-12 hours, for example 3-8 hours.

In one embodiment, in step (H-3-2), cooling to 0°C-30°C, for example to room temperature, is performed.

In one embodiment, in step (H-3-2), the cooling time is 0.5-30 hours, for example 12-24 hours.

In one embodiment, in step (H-3-2), the separation is carried out by centrifugal separation or filtration.

In one embodiment, the preparation method of the crystal form I comprises the following steps:
(I-1-1) suspending the compound of formula X in a solvent;
(I-1-2) mixing under suspension and centrifuging the mixture of step (I-1-1), or stirring under suspension the mixture of step (I-1-1) and then and separatingto obtain the crystal form I.

In one embodiment, in step (I-1-1), the solvent is water, n-heptane or methyl tert-butyl ether.

In one embodiment, step (I-1-1) and step (I-1-2) are carried out at room temperature.

In one embodiment, the preparation method of the crystal form I comprises the following steps:
(I-2-1) suspending the compound of formula X in a solvent;
(I-2-2) mixing and shaking under suspension, and then separating the mixture of step (I-2-1) to obtain the crystal form I.

In one embodiment, in step (I-2-1), the solvent is water, acetonitrile, isopropanol, acetone, ethyl acetate, tetrahydrofuran, n-heptane or methyl tert-butyl ether.

In one embodiment, step (I-2-2) is carried out at room temperature.

In one embodiment, in step (I-2-2), the time for shaking is 1-48 hours, for example 24-48 hours.

In one embodiment, the preparation method of the crystal form I comprises the following steps:
(I-3-1) suspending the compound of formula X in a solvent;
(I-3-2) mixing and shaking under suspension, and then separating the mixture of step (I-3-1) to obtain the crystal form I.

In one embodiment, in step (I-3-1), the solvent is acetonitrile, acetone, ethyl acetate or tetrahydrofuran.

In one embodiment, step (I-3-2) is carried out at 40°C-60°C, for example 50°C.

In one embodiment, in step (I-3-2), the time for shaking is 1-48 hours, for example 24-48 hours.

In one embodiment, the preparation method of the crystal form I comprises the following steps:
(I-4-1) dissolving the compound of formula X in a solvent;
(I-4-2) cooling and crystallizing the mixture of step (I-4-1), and then separating to obtain crystal form I.

In one embodiment, in step (I-4-1), the solvent is isopropanol, acetone or tetrahydrofuran.

In one embodiment, step (I-4-1) is carried out at 30°C-60°C, for example 40°C-60°C.

In one embodiment, step (I-4-2) is performed at 0°C to room temperature.

In one embodiment, the preparation method of the crystal form II comprises the following steps:
(II-1-1) dissolving the compound of formula X in a solvent;
(II-1-2) volatilizing the solution of step (II-1-1) slowly, and separating solid after precipitation to obtain the crystal form II.

In one embodiment, in step (II-1-1), the solvent is isopropanol.

In one embodiment, step (II-1-1) and step (II-1-2) are carried out at room temperature.

In one embodiment, the preparation method of the crystal form II comprises the following steps:
(II-2-1) suspending the compound of formula X in a solvent;
(II-2-2) mixing and shaking under suspension the mixture of step (II-2-1), and and separating to obtain the crystal form II.

In one embodiment, in step (II-2-1), the solvent is isopropanol.

In one embodiment, step (II-2-2) is carried out at 40°C-60°C, for example 50°C.

In one embodiment, in step (II-2-2), the time for shaking is 1-48 hours, for example 24-48 hours.

In one embodiment, the preparation method of the crystal form III comprises the following steps:
(III-1-1) suspending the compound of formula X in a solvent;
(III-1-2) shaking under suspension the mixture of step (III-1-1),and then separating to obtain the crystal form III.

In one embodiment, in step (III-1-1), the solvent is ethanol.

In one embodiment, step (III-1-2) is carried out at 20°C-60°C, for example room temperature to 50°C.

In one embodiment, in step (III-1-2), the time for shaking is 1-48 hours, for example 24-48 hours.

In one embodiment, the preparation method of crystal form III comprises the following steps:
(III-2-1) dissolving the compound of formula X in a solvent;
(III-2-2) cooling and crystallizing the mixture of step (III-2-1), and then separating to obtain crystal form III.

In one embodiment, in step (III-2-1), the solvent is ethanol.

In one embodiment, step (III-2-1) is carried out at 40°C-60°C.

In one embodiment, step (III-2-2) is carried out at 0°C to room temperature.

In a third aspect of the disclosure, there is provided a pharmaceutical composition comprising:
(a) the pharmaceutically acceptable salt of the compound of formula X, the polymorph of the compound of formula X, or the polymorph of pharmaceutically acceptable salt of the compound of formula X according to any one of the first aspect of the present disclosure; and (b) a pharmaceutically acceptable carrier.

In a fourth aspect of the disclosure, there is provided a use of the pharmaceutically acceptable salt of the compound of formula X, the polymorph of the compound of formula X, or the polymorph of pharmaceutically acceptable salt of the compound of formula X according to the first aspect of the present disclosure, or the pharmaceutical composition according to the third aspect of the present disclosure in the preparation of kinase inhibitor.

In one embodiment, the kinase inhibitor is a BTK inhibitor.

In a fifth aspect of the disclosure, there is provided a use of the pharmaceutically acceptable salt of the compound of formula X, the polymorph of the compound of formula X, or the polymorph of pharmaceutically acceptable salt of the compound of formula X according to the first aspect of the present disclosure, or the pharmaceutical composition according to the third aspect of the present disclosure in the preparation of a drug for the treatment and/or prevention of diseases mediated by B cell.

In a sixth aspect of the present disclosure, there is provided a method for treating diseases mediated by B cell, comprising administering to a patient in need thereof a therapeutically effective amount of the pharmaceutically acceptable salt of the compound of formula X, the polymorph of the compound of formula X, or the polymorph of pharmaceutically acceptable salt of the compound of formula X according to the first aspect of the present disclosure, or the pharmaceutical composition according to the third aspect of the present disclosure.

In one embodiment, the B cell-mediated disease is selected from a neoplastic disease, a proliferative disease, an allergic disease, an autoimmune disease, or an inflammatory disease.

In one embodiment, the B cell-mediated disease is selected from the group consisting of solid tumor, acute lymphocytic leukemia, chronic lymphocytic leukemia, acute myeloid leukemia, chronic myelogenous leukemia, rheumatoid arthritis, psoriatic arthritis, osteoarthritis, systemic lupus erythematosus, psoriasis, rheumatoid spine inflammation and gouty arthritis.

In one embodiment, the B cell-mediated disease is solid tumor.

In one embodiment, the solid tumor is at least one of lymphoma, soft tissue sarcoma, lymphocytic lymphoma, mantle cell lymphoma, melanoma and multiple myeloma.

It is to be understood that within the scope of the present disclosure, the various technical features of the present disclosure and the various technical features specifically described hereinafter (as in the embodiments) may be combined with each other to form a new or preferred technical solution. Due to space limitations, we will not repeat them here.

### BRIEF DESCRIPTION OF THE DRAWINGS

Figure 1-1 the X-ray powder diffraction pattern of the crystal form A
Figure 1-2 the thermogravimetric analysis spectrum of the crystal form A
Figure 1-3 the differential scanning calorimetry analysis spectrum of the crystal form A
Figure 1-4 the DVS pattern of the crystal form A
Figure 1-5 the polarized light microscope picture of the crystal form A
Figure 2-1 the X-ray powder diffraction pattern of the crystal form B-1
Figure 2-2 the X-ray powder diffraction pattern of the crystal form B-2
Figure 2-3 the X-ray powder diffraction pattern of the crystal form B-3
Figure 3 X-ray powder diffraction pattern of the crystal form C
Figure 4 the X-ray powder diffraction pattern of the crystal form D
Figure 5-1 the X-ray powder diffraction pattern of the crystal form E-1
Figure 5-2 the X-ray powder diffraction pattern of the crystal form E-2
Figure 6 the X-ray powder diffraction pattern of the crystal form F
Figure 7 the X-ray powder diffraction pattern of the crystal form G
Figure 8-1 the X-ray powder diffraction pattern of the crystal form H-1
Figure 8-2 the X-ray powder diffraction pattern of the crystal form H-2
Figure 8-3 the X-ray powder diffraction pattern of the crystal form H-3
Figure 9-1 the X-ray powder diffraction pattern of the crystal form I
Figure 9-2 the thermogravimetric analysis spectrum of the crystal form I
Figure 9-3 the differential scanning calorimetry analysis spectrum of the crystal form I

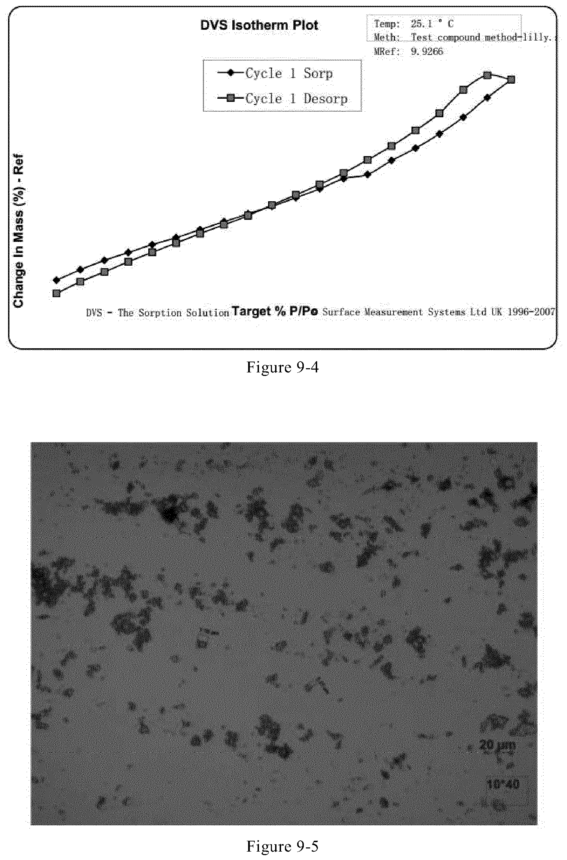

Figure 9-4 the DVS pattern of the crystal form I
Figure 9-5 the polarized light microscope picture of the crystal form I
Figure 10 the X-ray powder diffraction pattern of the crystal form II
Figure 11 the X-ray powder diffraction pattern of the crystal form III

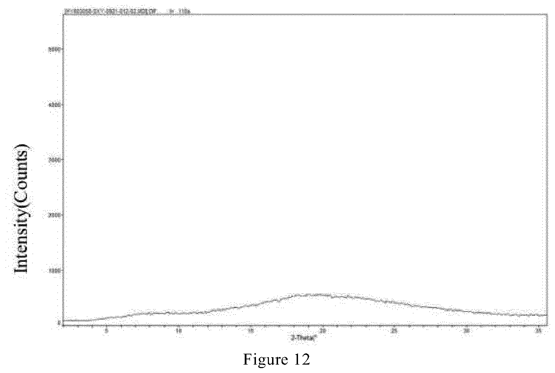

Figure 12 the X-ray powder diffraction pattern of the free base of compound of formula X in amorphous form

### DETAIL DESCRIPTION OF INVENTION

After extensive and in-depth research, the present inventors discovered a series of polymorphs of the free base of the compound of formula X, the salts of the compound of formula X, and the polymorphs of the salts of the compound of formula X, which have a high inhibitory activity against BTK. The study also found that a series of polymorphs of free base of the compound of formula X, the salts of the compound of formula X, and the polymorphs of the salts of the compound of formula X not only had good pharmacological activity *in vivo* and *in vitro,* but also had good physical and chemical stability, and therefore could be further developed into drugs.

### TERMS

As used herein, "the crystal of the present disclosure", "the crystal form of the present disclosure", "the polymorph of the present disclosure", etc. can be used interchangeably.

### Compound of formula X

In the present disclosure, the compound of formula X is (R)-6-((1-acryloylpiperidin-3-yl)amino)-7-fluoro-4-((2-fluoro-4-morpholinophenyl)amino)-1 ,2-di hydrogen-3H-pyrrolo[3,4-c]pyridin-3-one, which has a high inhibitory activity against BTK WT kinase and βBTK Y223 cells, but weak inhibitory activity against wild-type EGFR kinase. Therefore, it has selective inhibitory activity on BTK WT kinase.

The disclosure also includes a pharmaceutically acceptable salt of the compound of formula X, a polymorph of the compound of formula X, or a polymorph of pharmaceutically acceptable salt of the compound of formula X.

In the present disclosure, the pharmaceutically acceptable salt is selected from the group consisting of hydrochloride, sulfate, hydrobromide, phosphate, methanesulfonate, maleate, L-tartrate, citrate, fumarate, and succinate.

### Polymorphs

Solid exists in amorphous form or in crystalline form. In the case of crystal form, the molecules are localized in the three-dimensional lattice sites. When a compound is crystallized from a solution or slurry, it can be crystallized in different space lattice arrangement (this property is called "polymorphism") to form crystals with different crystalline forms, each of which is called as "polymorphs." Different polymorphs of a given substance may differ from each other in one or more physical properties (such as solubility and dissolution rate, true specific gravity, crystalline form, packing pattern, flowability and/or solid state stability).

### Crystallization

Crystallization at the production scale can be accomplished by manipulating a solution such that the solubility limit of the interested compound is exceeded. This can be done in a number of ways, for example, by dissolving the compound at a relatively high temperature and then cooling the solution below a saturation limit, or by boiling, evaporating at ordinary pressure, drying in vacuo or by some other means to reduce the liquid volume. The solubility of the interested compound may be reduced by adding an anti-solvent or a solvent in which the compound has a low solubility or a mixture of such solvents. An alternative method is to adjust the pH to reduce the solubility. See Crystallization, Third Edition, J W Mullens, Butterworth-Heineman Ltd., 1993, ISBN 0750611294 for a detailed description of crystallization.

The "stirring under suspension" described in the present disclosure means a way to get crystals by mixing the compound of formula X with a corresponding acid or a solution of the corresponding acid in a suitable solvent to form a turbid solution, or by mixing the compound of formula X with a suitable solvent to form a turbid solution before stirring. The suitable solvent may be water or an organic solvent.

The "slow volatilization" described in the present disclosure means a way to get crystals by leaving a solution of the compound of formula X or a solution containing the compound of formula X and the corresponding acid at a certain temperature for slow volatilization of the solvent.

The "addition of anti-solvent" or "adding anti-solvent" described in the present disclosure means a way to get crystals by adding a suitable solvent to a solution of the compound of formula X and precipitating the crystals.

If salt formation and crystallization are expected to occur at the same time, the addition of an appropriate acid or base can result in the direct crystallization of the desired salt if the salt has a lower solubility in the reaction medium than the raw material. Likewise, in a medium in which the solubility of the desired final form is lower than that of reactant, the final product can be directly crystallized when the synthetic reaction is completed.

Optimization of crystallization can include inoculation of the crystal of desired form as a seed into the crystallization medium. In addition, many crystallization methods include a combination of the above strategies. One example is to dissolve the interested compound in a solvent at a high temperature, followed by the addition of an anti-solvent at a suitable volume in a controlled manner so that the system is just below saturation level. At this moment, the seed of desired form (the integrity of the seed is kept) can be added and the system is cooled to complete the crystallization.

As used herein, the term "room temperature" generally means 4 °C -30 °C, for example 20 ± 5 °C.

### Polymorphs of the present disclosure

As used herein, the term "the polymorph of the present disclosure" includes the polymorph of the compound of formula X or the polymorph of the pharmaceutically acceptable salts of the compound of formula X (e.g. hydrochloride, fumarate), or the polymorph of various solvates of the compound of formula X, and also includes different polymorphs of the same salt or solvate.
"compound of formula X" and "the free base of the compound of formula X" can be used interchangeably, and "the polymorph of the compound of formula X" and "the polymorph of the free base of the compound of formula X" can be used interchangeably.

Polymorphs of the disclosure include (but are not limited to):
(i) crystal forms A, B-1, B-2, B-3, C, D, E-1, E-2, F, G, H-1, H-2, H-3 (the polymorph of salt of the compound of formula X);
(ii) crystal forms I, II, III (the polymorph of the compound of formula X).

In the present disclosure, some crystal forms can be converted into each other, so the present disclosure also provides a method of converting some crystal forms into each other.

### Identification and properties of polymorphs

The properties of the polymorph of the compound of formula X were studied using the following various ways and instruments after its preparation in the present disclosure.

### X-ray powder diffraction

Methods of determining X-ray powder diffraction of the crystals are known in the art. For example, an X-ray powder diffractometer was used to obtain a pattern with a copper radiation target at a scanning speed of 2° per minute.

The polymorph of the compound of formula X of the present disclosure or the polymorph of the pharmaceutically acceptable salt of the compound of formula X of the present disclosure has a specific crystalline form and has specific characteristic peaks in an X-ray powder diffraction (XRPD) pattern.

### Differential scanning calorimetry

It is also called "differential scanning calorimetry analysis" (DSC) which is a technique that measures the relationship between energy difference between the measured substance and the reference substance and temperature during heating. The location, shape and number of peaks on the DSC pattern are related to the nature of the substance, and therefore can be used to qualitatively identify the substance. This method can be commonly used in the art to detect the phase transition temperature, glass transition temperature, reaction heat and other parameters of a substance.

### Pharmaceutical compositions of compound of formula X and their use

In general, the compound of formula X of the present disclosure, or a polymorph of the pharmaceutically acceptable salt thereof, can be administered in a suitable dosage form with one or more pharmaceutically acceptable carriers. These dosage forms are suitable for oral, rectal, topical, intraoral, and other parenteral administration (e.g., subcutaneous, intramuscular, intravenous, etc.). For example, dosage forms suitable for oral administration include capsules, tablets, granules, syrups, and the like. The compound of the present disclosure contained in these preparations may be a solid powder or granule, a solution or suspension in an aqueous or non-aqueous liquid, a water-in-oil or oil-in-water emulsion or the like. The above dosage forms can be prepared from the active compound with one or more carriers or excipients via conventional pharmaceutical methods. The above carriers need to be compatible with the active compound or other excipients. For solid preparations, commonly used non-toxic carriers include, but not limited to, mannitol, lactose, starch, magnesium stearate, cellulose, glucose, sucrose, and the like. Carriers for liquid preparations include water, physiological saline, glucose aqueous solution, ethylene glycol, polyethylene glycol, and the like. The active compound can form a solution or suspension with the above carriers.

The compositions of the present disclosure are formulated, quantified, and administered in a manner consistent with medical practices. The "effective amount" of a given compound will be determined by the factors such as the particular condition to be treated, the individual to be treated, the cause of the condition, the target of the drug, the mode of administration and the like.

The present disclosure provides a pharmaceutically acceptable salt of the compound of formula X, a polymorph of the compound of formula X or a polymorph of the pharmaceutically acceptable salt of the compound of formula X according to the first aspect of the present disclosure for use in the preparation of a BTK inhibitor or a medicament for treating BTK related diseases.

For example, the BTK related diseases are cancer, abnormal cell proliferative diseases, infections, inflammatory disorders, autoimmune diseases, cardiovascular diseases, neurodegenerative diseases, radiation-induced hematopoietic toxic diseases, or a combination thereof.

For example, the cancer is breast cancer, ovarian cancer, prostate cancer, melanoma, brain tumor, esophageal cancer, stomach cancer, liver cancer, pancreatic cancer, colorectal cancer, lung cancer, kidney cancer, skin cancer, glioblastoma, neuroblastoma, sarcoma, liposarcoma, osteochondroma, osteoma, osteosarcoma, seminoma, testicular tumor, uterine cancer, head and neck tumor, multiple myeloma, malignant lymphoma, polycythemia vera, leukemia, thyroid tumors, ureteral tumor, bladder tumor, gallbladder cancer, cholangiocarcinoma, chorionic epithelial cancer or pediatric tumors, or any combination thereof.

For example, the breast cancer is HR-positive, HER2-negative advanced breast cancer.

As used herein, "therapeutically effective amount" refers to the amount that has a function or activity to humans and/or animals and may be tolerated by humans and/or animals.

As used herein, "pharmaceutically acceptable carrier" refers to a non-toxic, inert, solid, semi-solid substance or liquid filler, diluent, encapsulating material or auxiliary formulation or any type of excipient that is compatible with the patient which is preferably a mammal, for example a human. It is suitable for delivering active agent to a target without stopping the activity of the agent.

As used herein, "patient" refers to an animal, preferably a mammal, and more preferably a human. The term "mammal" refers to a warm-blooded vertebrate mammal, including, for example, cat, dog, rabbit, bear, fox, wolf, monkey, deer, rat, pig and human.

As used herein, "treatment" refers to alleviating, delaying progression, attenuating, preventing, or maintaining an existing disease or disorder (eg, cancer). Treatment also includes curing one or more symptoms of the disease or disorder, preventing its development or alleviating to some extent.

The therapeutically effective amount of the pharmaceutical composition of the present disclosure or the pharmaceutically acceptable salt of the compound of formula X, the polymorph of the compound of formula X or the polymorph of pharmaceutically acceptable salt of the compound of formula X contained in the pharmaceutical composition can be 0.1 mg- 5g/kg (body weight).

### The main advantages of the present disclosure are:

The inventor found that the polymorphs of free base and salts thereof (R)-6-((1-acryloylpiperidin-3-yl)amino) -7-fluoro-4-((2-fluoro-4-morpholinophenyl)amino)-1,2-dihydrogen-3H-pyrrolo[3,4-c]pyridin-3-o ne, and the salts of (R)-6-((1-acryloylpiperidin-3-yl)amino) -7-fluoro-4-((2-fluoro-4-morpholinophenyl)amino)-1,2-dihydrogen-3H-pyrrolo[3,4-c]pyridin-3-o ne also had good physical and chemical stability and outstanding related pharmacological activities, and was an ideal BTK inhibitor.

### DETAILED DESCRIPTION OF THE INVENTION

The present disclosure will be further illustrated below with reference to the specific examples. It should be understood that these examples are only to illustrate the disclosure but not to limit the scope of the present disclosure. The experimental methods without specific conditions in the following embodiments are generally carried out according to conventional conditions, or in accordance with the conditions recommended by the manufacturer. Unless indicated otherwise, parts and percentage are calculated by weight.

### Reagents and Instruments

In the present disclosure, the structure and purity of the compounds are identified by nuclear magnetic resonance (¹HNMR) and, or LC-MS mass spectrometry (LC-MS). ¹HNMR: BrukerAVANCE-400 NMR machine, with tetramethylsilane (TMS) as the internal standard. LC-MS: Agilent 1200 HPLC System, 6140 MS liquid-mass spectrometer (available from Agilent), column WatersX-Bridge, 150×4.6mm, 3.5µm. Preparative high performance liquid chromatography (pre-HPLC): Waters PHW007, column XBridge C18, 4.6^{∗}150mm, 3.5um.

ISCO Combiflash-Rf75 or Rf200 automatic eluting column instrument, and disposable Agela silica gel column (4 g, 12g, 20g, 40g, 80g, 120g) were used.

Yantai Huanghai HSGF254 or Qingdao GF254 silica gel plates was used as thin-layer silica gel plate, in thin layer chromatography (TLC), 0.15mm - 0.2mm silica gel plates were used for detection of reaction, 0.4mm - 0.5mm silica gel plates were used for separation and purification of products in TLC. Yantai Huanghai 200-300 mesh silica gel is generally used as a carrier. FCP200 - 300 mesh alkaline aluminum oxide for medicine chromatography in China is commonly used as a carrier in alkaline aluminum oxide column.

All reactions were performed under nitrogen or argon atmosphere and the solution refers to the aqueous solution if without special explanation in the examples.

As used herein, DCM refers to dichloromethane, DMF refers to dimethylformamide, DMSO refers to dimethylsulfoxide, THF refers to tetrahydrofuran, DIPA refers to diisopropylamine, DIPEA or DIEA refers to N,N-diisopropylethylamine, NMP refers to N-methylpyrrolidone, n-BuLi refers to n-butyl lithium, NaBH(OAc)₃ refers to sodium triacetoxyborohydride, Xantphos refers to 4,5-bis(diphenylphosphine)-9,9-bis methylxanthene, Xphos refers to 2-dicyclohexylphosphorus-2',4',6'-triisopropylbiphenyl, TFA refers to trifluoroacetic acid, EA refers to ethyl acetate, PE refers to petroleum ether, BINAP refers to (2R, 3S)-2,2'- bis diphenylphosphino-1,1'-binaphthyl, NBS refers to N-bromosuccinimide, NCS refers to N-chlorosuccinimide, Pd₂(dba)₃ refers to tris(dibenzylideneacetone)dipalladium, and Pd(dppf)Cl2 refers to [1,1'-bis (diphenylphosphino) ferrocene] palladium dichloride, and Et₃SiH refers to triethylsilane.

Acetonitrile ACN, methanol MeOH, ethanol EtOH, isopropyl alcohol IPA, acetone ACE, ethyl acetate EA, methyl tert-butyl ether MTBE, tetrahydrofuran THF, water H₂O, 50% acetonitrile 50% ACN.

As used herein, room temperature refers to about 20 ± 5 °C.

### General method

X-ray powder diffraction: in the present disclosure, the powder X-ray diffraction patterns of the above crystal forms are obtained by a method known in the art, using a D8 ADVANCE X-ray powder diffraction analyzer. The instrument test conditions are shown in the following table:

| Parameter | XRPD(Reflection mode) |
|---|---|
| X-ray | Cu, kα, Kα1 (Å): 1.54056 |
| X-ray tube settings | 40 kV, 40 mA |
| divergence slit | automatic |
| monochromator | none |
| Scanning mode | continuous |
| Scanning range (°2Theta) | 4°-40° |
| Scanning step (°2Theta) | 0.013 |
| Scan time (minutes) | ∼7 |

In the powder X-ray diffraction pattern, the site of each peak was determined by 2θ(°). It should be understood that different instruments and/or conditions could result in slightly different data and changes in peak site and relative intensity. The division of the intensity of peaks only reflects the approximate size of peaks in each site. In the present disclosure, the highest diffraction peak of each crystalline form was taken as the base peak which was defined as I₀ with the relative intensity as 100%, (the peak of crystal form I with 2θ(°) value of 20.27 is the base peak, the peak of crystal form II with 2θ(°) value of 7.32 is the base peak, the peak of crystal form III with 2θ(°) value of 17.83 is the base peak, the peak of crystal form A with 2θ(°) value of 26.21 is the base peak, the peak of crystal form B-1 with 2θ(°) value of 17.59 is the base peak, the peak of crystal form B-2 with 2θ(°) value of 26.32 is the base peak, the peak of crystal form B-3 with 2θ(°) value of 17.03 is the base peak, the peak of crystal form C with 2θ(°) value of 25.87 is the base peak, the peak of crystal form D with 2θ(°) value of 26.38 is the base peak, the peak of crystal form E-1 with 2θ(°) value of 17.80 is the base peak, the peak of crystal form E-2 with 2θ(°) value of 17.80 is the base peak, the peak of crystal form F with 2θ(°) value of 18.58 is the base peak, the peak of crystal form G with 2θ(°) value of 28.48 is the base peak, the peak of crystal form H-1 with 2θ(°) value of 21.70 is the base peak, the peak of crystal form H-2 with 2θ(°) value of 19.78 is the base peak, the peak of crystal form H-3 with 2θ(°) value of 25.82 is the base peak), and other peaks had the ratio of their peak height to the peak height of base peak as the relative intensity I/I₀. The definition of the relative intensity of each peak was shown in the following table:

| Relative intensity I/I₀(%) | Definition |
|---|---|
| 50-100 | VS (very strong) |
| 25-50 | S (strong) |
| 10-25 | M (medium) |
| 1-10 | W (weak) |

The acid-base molar ratio of the salts of the present disclosure or their crystalline forms was determined by HPLC/ IC or ¹H NMR.

High performance liquid chromatography: in the present disclosure, high performance liquid chromatography (HPLC) is collected on an Agilent 1260 HPLC.

TGA and DSC pattern: TGA and DSC pattern were collected on TA Q500/5000 thermogravimetric analyzer and TA Q200/2000 differential scanning calorimeter respectively. The instrument test conditions are shown in the following table:

| Parameter | TGA | DSC |
|---|---|---|
| Method | Linear warming | Linear warming |
| Sample tray | Platinum plate, open | Aluminum plate, gland |
| Temperature range | Room temperature - set temperature | 25 °C - set temperature |
| Scanning rate (°C/ min) | 10 | 10 |
| Protective gas | Nitrogen | Nitrogen |

The Dynamic Vapor Sorption (DVS) curve: acquired on the DVS Intrinsic of Surface Measurement Systems. The relative humidity at 25 ° C is corrected with the deliquescence points of LiCl, Mg (NO₃)₂ and KCl. The instrument test conditions are shown in the following table:

| Parameters | Setting value |
|---|---|
| Temperature | 25 °C |
| Sample size | 10-20 mg |
| Protective gas and flow | N₂, 200 ml/min |
| dm/dt | 0.002%/min |
| Minimum dm/dt balance time | 10 minutes |
| The maximum balance time | 180 minutes |
| RH range | 0%RH-90%RH-0%RH |
| RH gradient | 10%(0%RH-90%RH, 90%RH-0%RH) 5%(90%RH-95%RH, 95%RH-90%RH) |

It should be understood that other numerical values may be obtained when other types of instruments with the same function as the instruments described above or test conditions which are different from the conditions used in the present disclosure were used. Therefore, the recited value should not be considered as an absolute numerical value.

Due to the instrumental errors or different operators, one skilled in the art will understand that the above parameters used to characterize the physical properties of crystals may differ slightly, so the parameters described above are only used to assist in characterizing the polymorphs provided herein, and can not be regarded as a limitation on the polymorphs of the present disclosure.

### Preparation of intermediate 1a

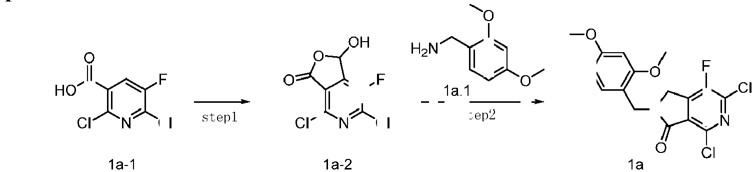

Step 1: n-BuLi (27mL, 66mmol) and DIPA (6.6g, 66mmol) were added to a solution of compound 1a-1 (6.0g, 30.0mmol) in THF (80mL) at -78°C, the mixture was stirred for 1h and then DMF (10mL) was added, the mixture was warmed to room temperature and stirred for another 2 h. The reaction was followed by LC-MS until it was completed. The mixture was adjusted to pH 5-6 by adding HCl (2N) to the system, and extracted with ethyl acetate. The organic phase was washed with saturated brine, dried over anhydrous sodium sulfate and concentrated, purified by column chromatography to give 6.8 g of compound 1a-2. MS m/z (ESI): 238 [M+H]⁺.

Step 2: Compound 1a.1 (15g, 90.0mmol), acetic acid (2mL) and NaBH(OAc)₃ (18.9g, 90.0mmol) were added to a solution of compound 1a-2 (6.8g, 30.0mmol) in 1,4-dioxane (80mL), and then the mixture was stirred at 50 °C overnight. The reaction was followed by LC-MS until it was completed. The reaction solution was evaporated to dryness under reduced pressure, washed with saturated brine and extracted with DCM. The organic phase was dried and concentrated, purified by column chromatography to give 4.8 g of compound 1a. MS m/z (ESI): 371 [M+H]⁺.

### Preparation of intermediate 1b

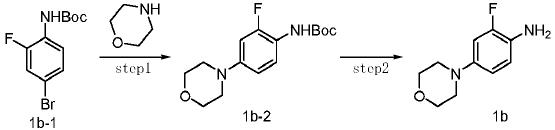

Step 1: A solution of compound 1b-1 (1.5g, 5.17mmol), morpholine (470mg, 5.39mmol), Pd₂(dba)₃ (210mg, 0.23mmol), Xphos(240mg, 0.503mmol) and cesium carbonate (3.38g, 10.37mmol) in 1,4-dioxane (20mL) was added to a 100mL three-necked flask, the reaction solution was reacted at 110°C for 3h, followed by LC-MS until it was completed. The reaction solution was cooled to room temperature, concentrated and purified by column chromatography (n-hexane containing 0-20% EA) to obtain a gray solid compound 1b-2 (1.38 g, yield:90.1%). MS m/z(ESI): 297.2[M+H]⁺.

Step 2: Compound 1b-2 (1.38g, 4.657mmol), methanol (20mL) and HCl/1,4-dioxane (4M, 10mL) were added to a 100mL flask. The mixture was stirred overnight at room temperature. The reaction solution was concentrated under reduced pressure to remove the solvent, the residue was washed with saturated sodium bicarbonate, extracted with dichloromethane, and the organic layer was dried and concentrated to obtain 900 mg of yellow solid compound 1b. MS m/z(ESI): 197.2[M+H]⁺.

### Example 1 Preparation of compound of formula X

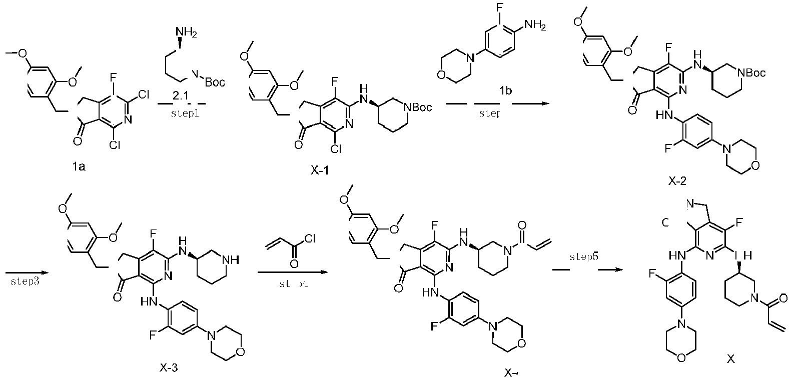

Step 1: Compound 2.1 (600mg, 3mmol) and DIPEA (780mg, 6mmol) were added to a solution of compound 1a (740mg, 2mmol) in NMP (10 mL), the mixture was reacted in microwave at 180°C for 30min under argon atmosphere. The reaction was followed by LC-MS until it was completed. The reaction solution was cooled to room temperature, diluted with DCM, washed with water and saturated brine, the organic layer was dried, concentrated and purified by column chromatography to obtain 300 mg of compound X-1. MS m/z(ESI): 535[M+H]⁺.

Step 2: A solution of compound X-1 (250mg, 0.5mmol), compound 1b (118mg, 0.6mmol), Pd₂(dba)₃ (45mg, 0.05mmol), Xantphos (54mg, 0.1mmol) and cesium carbonate (326mg, 1mmol) in 1,4-dioxane (15 mL) was reacted in microwave at 160°C for 50min under argon atmosphere. The reaction was followed by LC-MS until it was completed. The reaction solution was cooled to room temperature, diluted with EA, washed with water and saturated brine, the organic layer was dried, concentrated and purified by column chromatography to obtain 185 mg of compound X-2. MS m/z(ESI): 695.3[M+H]⁺.

Step 3: TFA (4.5mL) was added to a solution of compound X-2 (185mg, 0.27mmol) in DCM (12mL). The mixture was stirred at room temperature for 1, followed by LC-MS until it was completed. Most of TFA was removed under reduced pressure, and saturated sodium bicarbonate was added. The solution was adjusted to pH 7-8, extracted with DCM, the organic layers were combined and dried, and concentrated to obtain compound X-3, which was directly used in the next reaction. MS m/z(ESI): 595.2[M+H]⁺.

Step 4: Acryloyl chloride (15.4mg, 0.17mmol) and DIPEA (66mg, 0.51mmol) were added to a solution of compound X-3 (100mg) in DCM (10mL) under argon atmosphere. The mixture was stirred at room temperature for 2h. The reaction was followed by LC-MS until it was completed. The reaction solution was washed with saturated brine, extracted with DCM, the organic layer was dried, and concentrated to obtain a crude product, which was purified by column chromatography to obtain 86 mg of compound X-4. MS m/z(ESI): 649[M+H]⁺.

Step 5: Et₃SiH (0.2mL) was added to a solution of compound X-4 (86mg, 0.13mmol) in TFA (3mL), and the mixture was heated to 80°C and stirred for 2h. The reaction was followed by LC-MS until it was completed. Most of TFA was removed under reduced pressure, and the solution was adjusted the pH to 7-8 with saturated sodium bicarbonate solution, extracted with ethyl acetate, the organic layers were combined, dried and concentrated, and then purified by Prep-HPLC (eluent: DCM:MeOH=100:3) to obtain 10mg of solid compound X. MS m/z(ESI):499.3[M+H]⁺. ¹H NMR (400 MHz, DMSO-*d*₆) δ 8.65 (d, *J* = 2.6 Hz, 1H), 8.28-8.16 (m, 2H), 7.08 - 6.99 (m, 1H), 6.87 (d, *J =* 14.3 Hz, 1.5H), 6.58 (d, *J=* 9.1 Hz, 1.5H), 6.18-5.98 (m, 1H), 5.72 (d, *J* = 10.4 Hz, 0.5H), 5.42 (d, *J* = 10.6 Hz, 0.5H), 4.55 (d, *J =* 12.4 Hz, 0.5H), 4.37 (s, 2H), 4.17 (d, *J =* 12.6 Hz, 0.5H), 3.99 (s, 1H), 3.89 (s, 1H), 3.71 (t, *J =* 4.6 Hz, 4H), 3.03-2.98 (m, 5H), 2.66-2.85(m, 1H), 2.00(s, 1H), 1.79 (d, *J* = 13.5 Hz, 1H), 1.65 (d, *J* = 12.5 Hz, 1H), 1.43 (s, 1H). The obtained solid was sent for XRD detection, and its powder X-ray diffraction pattern showed no characteristic peaks, the powder X-ray diffraction pattern is shown in Figure 12 (the 2θ angle has been marked), so it was in amorphous form.

### Example 2 Preparation of crystal form I of the compound of formula X

An appropriate amount of the free base of the compound of formula X (amorphous) was weighed into a sample bottle, and an appropriate amount of the solvents in the table below was added gradually to the bottle while stirring at room temperature to obtain a suspension of the compound of formula X, and the suspension was centrifuged and dried to obtain a solid, and the solid was sent for XRD detection. The powder X-ray diffraction pattern of the obtained solid is shown in Figure 9-1 (the 2θ angle has been marked), which is defined as crystal form I in this application. The added free base of the compound of formula X, the type and amount of solvent, the preparation method and the obtained crystal type are shown in table 1:

**Table 1**

| Solvent | Addition amount of the free base of the compound of formula X mg | Addition amount of the solvent in ml | Preparation method | Crystal form |
|---|---|---|---|---|
| water | 10.17 | 3 | mixing under suspension, centrifugation and drying | crystal form I |
| methyl tert-butyl ether | 10.07 | 1 | mixing under suspension, centrifugation and drying | crystal form I |
| n-heptane | 10.00 | 1 | mixing under suspension, centrifugation and drying | crystal form I |

### Example 3 Preparation of crystal form I of the compound of formula X

20mg of the free base of the compound of formula X(amorphous) was weighed into a centrifuge tube respectively, an appropriate amount of solvent was added and heated to 50°C until dissolved, after cooling down, a solid is precipitated and separated, and then sent for XRD detection. The powder X-ray diffraction pattern of the obtained solid is shown in Figure 9-1 (the 2θ angle has been marked), which is defined as crystal form I in this application. The solvent used and the corresponding crystal form obtained are shown in table 2:

**Table 2**

| Solvent/preparation method/crystal form | Crystal form |
|---|---|
| isopropanol | crystal form I |
| acetone | crystal form I |
| tetrahydrofuran | crystal form I |

### Example 4 Preparation of crystal form I of the compound of formula X

20mg of the free base of the compound of formula X(amorphous) was weighed into a centrifuge tube respectively, an appropriate amount of solvent was added to make the compound suspended in the solvent, the suspension was shaked at room temperature for 1 day, the solid was separated, and sent for XRD detection. The powder X-ray diffraction pattern of the solid is shown in Figure 9-1 (the 2θ angle has been marked), which is defined as crystal form I in this application. In addition, 20 mg of the compound of formula X was weighed into a centrifuge tube respectively, and an appropriate amount of solvent was added to make the compound suspended in the solvent, the suspension was shaked at 50°C for 1 day, the solid was separated, and sent for XRD detection. The powder X-ray diffraction pattern of the obtained solid is shown in Figure 9-1 (the 2θ angle has been marked), that is crystal form I. The TGA pattern of crystal form I is shown in Figure 9-2. According to the TGA pattern, crystal form I has basically no weight loss below 250°C and has good stability. The DSC and DVS patterns of crystal form I are respectively as shown in Figure 9-3 and Figure 9-4, the crystal form I has 0.22% of weight gain of moisture absorption at 90% RH, which is slightly hygroscopic. The polarized microscope picture of the crystal form I is shown in Figure 9-5. The crystal form I is granular with a very small particle size of about a few microns. The solvent used and the corresponding crystal form obtained are shown in table 3 and table 4:

**Table 3**

| Solvent/preparation method/ crystal form | mixing and shaking under suspension (room temperature) |
|---|---|
| water | crystal form I |
| acetonitrile | crystal form I |
| isopropanol | crystal form I |
| acetone | crystal form I |
| ethyl acetate | crystal form I |
| methyl tert-butyl ether | crystal form I |
| tetrahydrofuran | crystal form I |
| n-heptane | crystal form I |

**Table 4**

| Solvent/preparation method/crystal form | mixing and shaking under suspension (50°C) |
|---|---|
| acetonitrile | crystal form I |
| acetone | crystal form I |
| ethyl acetate | crystal form I |
| tetrahydrofuran | crystal form I |

### Example 5 Preparation of crystal form II of the free base of compound of formula X

10.44mg of the free base of the compound of formula X (amorphous) was weighed into a sample bottle, 2.5ml isopropanol was added gradually to the bottle at room temperature to make the compound completely dissolved, the solution was placed in a fume hood for slow volatilization to precipitate solids, and send the solids for XRD detection. The powder X-ray diffraction pattern of the resulting solid is shown in Figure 10 (the 2θ angle has been marked), which is defined as crystal form II in this application. The study found that the XRD patterns of the solids prepared by the same method using acetone, acetonitrile, ethyl acetate, ethanol, methanol, tetrahydrofuran and dimethyl sulfoxide as solvents showed no characteristic peaks and were in an amorphous form.

### Example 6 Preparation of crystal form II of the free base of compound of formula X

20 mg of the free base of the compound of formula X (amorphous) was weighed into a centrifuge tube, an appropriate amount of isopropanol was added to obtain a suspension. The suspension was shaked at 50 °C for 1 day, the solid was separated and sent for XRD detection. The powder X-ray diffraction pattern of the resulting solid is shown in Figure 10 (the 2θ angle has been marked), which is defined as crystal form II in this application.

### Example 7 Preparation of crystal form III of the free base of compound of formula X

20mg of the free base of the compound of formula X (amorphous) was weighed into a centrifuge tube respectively, an appropriate amount of ethanol was added to make the compound suspended in the solvent, one sample was shaked at room temperature for 1 day, and the other one was shaked at 50°C for 1 day. The solid was separated and sent for XRD detection. The powder X-ray diffraction pattern of the obtained solid is shown in Figure 11 (the 2θ angle has been marked), which is defined as crystal form III in this application. The results are shown in table 5.

**Table 5**

| Solvent | mixing and shaking under suspension ( room temperature) | mixing and shaking under suspension (50°C) |
|---|---|---|
| ethanol | crystal form III | crystal form III |

### Example 8 Preparation of crystal form III of the free base of compound of formula X

20mg of the free base of the compound of formula X(amorphous) was weighed into a centrifuge tube, an appropriate amount of ethanol was added and heated to 50°C until dissolved, after cooling down, a solid is precipitated and separated, and then sent for XRD detection. The powder X-ray diffraction pattern of the obtained solid is shown in Figure 11 (the 2θ angle has been marked), which is defined as crystal form III in this application.

### Example 9 Preparation of crystal form A of the compound of formula X

20mg of the free base of the compound of formula X (amorphous) was weighed into each sample bottle, an appropriate amount of the corresponding organic solvent was added respectively, after the compound was dissolved, 50µL of 1M hydrochloric acid was added to conduct a salt-forming reaction. A magnetic stir bar was added to the sample bottle and the solution was reacted for 4 hours under a 50°C water bath using a magnetic stirrer, the reaction was carried out while heating and stirring. After the reaction was completed, the reaction solution was cooled to room temperature and became turbid, the turbid liquid was centrifuged, and then dried and sent for XRD test. The powder X-ray diffraction pattern of the obtained crystal is shown in Figure 1-1 (the 2θ angle has been marked), and the acid-base molar ratio is 1.2:1. It is defined as crystal form A in this application. The TGA pattern of crystal form A is shown in Figure 1-2. According to the TGA pattern, the weight loss of crystal form A was 5.9% during the heating process from about 50°C to 100°C, which may be due to the volatilization of organic solvent and the removal of hydrochloric acid, the weight of crystal form A is further reduced from 100°C to 160°C. It may be due to the decomposition of the compound, and the hydrochloride is unstable at high temperatures. The DSC spectrum and DVS spectrum of crystal form A are shown in Figures 1-3 and Figure 1-4, respectively, the crystal form A has 7.509% of weight gain of moisture absorption at 90% RH, and is hygroscopic. The polarized microscope picture of crystal form A is shown in Figure 1-5. The crystal form A is granular with a very small particle size of about a few microns. The crystal forms formed by various solvents are shown in table 6.

**Table 6**

| | | | | |
|---|---|---|---|---|
| Acid | isopropanol | acetone | ethyl acetate | acetonitrile |
| hydrochloric acid | amorphous | crystal form A | crystal form A | crystal form A |

### Example 10 Preparation of crystal form A of the compound of formula X

The free base of the compound of formula X (amorphous) was weighed according to the following table, and dissolved or suspended in ethyl acetate or acetone, the hydrochloric acid with the corresponding concentration was added slowly according to the acid-base molar ratio of 1.2:1 to conduct salt formation reaction, the temperature of water bath is 50°C, and the reaction time is 4-8h. The heating is stopped after the reaction, the sample is slowly cooled to room temperature in a water bath, the reaction liquid becomes turbid, the turbid liquid sample is centrifuged (8000rpm, 10min) in a centrifuge. The supernatant is discarded, and the residue is dried. The powder X-ray diffraction pattern of the obtained crystal is shown in Figure 1-1 (the 2θ angle has been marked), and the acid-base molar ratio is 1.2:1. It is defined as crystal form A in this application. The reaction conditions are shown in table 7:

**Table 7**

| Sample | State before adding acid | Cooling time | Concentration of hydrochloric acid | Solvent |
|---|---|---|---|---|
| 20mg | Clear | Not overnight | 1M | ethyl acetate |
| 40mg | Turbid | overnight | 0.25M | ethyl acetate |
| 20mg | Clear | overnight | 0.25M | ethyl acetate |
| 20mg | Clear | overnight | 0.5M | ethyl acetate |
| 20mg | Clear | overnight | 0.75M | ethyl acetate |
| 40mg | Clear | overnight | 1M | ethyl acetate |
| 100mg | Turbid | overnight | 1M | ethyl acetate |
| 10mg | Clear | overnight | 1M | ethyl acetate |
| 200mg | Turbid | overnight | 1M | ethyl acetate |
| 100mg | Clear | overnight | 1M | ethyl acetate |
| 50mg | Clear | overnight | 1M | ethyl acetate |
| 50mg | Clear | overnight | 1M | acetone |
| 100mg | Clear | overnight | 1M | acetone |

### Example 11 Preparation of crystal form G of the compound of formula X

20mg of the free base of the compound of formula X (amorphous) was weighed into each sample bottle, and an appropriate amount of the corresponding organic solvent was added. After the compound was dissolved, 200µL of 0.25M fumaric acid was added to conduct a salt-forming reaction. A magnetic stir bar was added to the sample bottle and the solution was reacted for 4 hours under a 50°C water bath using a magnetic stirrer, the reaction was carried out while heating and stirring. After the reaction was completed, the reaction solution was cooled to room temperature and became turbid, the turbid liquid was centrifuged, and then dried and sent for XRD test. The powder X-ray diffraction pattern of the obtained crystal is shown in Figure 7 (the 2θ angle has been marked), and the acid-base molar ratio is 1.2:1. It is defined as crystal form G in this application. The crystal forms formed by various corresponding solvents are shown in table 8.

**Table 8**

| | | | | |
|---|---|---|---|---|
| Acid | isopropanol | acetone | ethyl acetate | acetonitrile |
| fumaric acid | crystal form G | crystal form G | crystal form G | crystal form G |

### Example 12 Preparation of crystal form G of the compound of formula X

The free base of the compound of formula X (amorphous) was weighed according to the following table, and dissolved or suspended in ethyl acetate or tetrahydrofuran, the fumaric acid with the corresponding concentration was added slowly according to the acid-base molar ratio of 1.2:1 to conduct a salt-forming reaction, the temperature of water bath is 50°C, and the reaction time is 4-8h. The heating is stopped after the reaction, the sample is slowly cooled to room temperature in a water bath, the reaction liquid becomes clear, and the clear liquid is dried. The powder X-ray diffraction pattern of the obtained crystal is shown in Figure 7 (the 2θ angle has been marked), and the acid-base molar ratio is 1.2:1. It is defined as crystal form G in this application. The reaction conditions are shown in table 9:

**Table 9**

| Sample | State before adding acid | Concentration of fumaric acid | Solvent | Cooling time |
|---|---|---|---|---|
| 20mg | Clear | 0.25M | ethyl acetate | Not overnight |
| 40mg | Turbid | 0.25M | ethyl acetate | overnight |
| 20mg | Clear | 0.5M | ethyl acetate | overnight |
| 20mg | Clear | 0.5M | tetrahydrofuran | overnight |
| 20mg | Turbid | 1M | ethyl acetate | overnight |
| 20mg | Clear | 0.5M | ethyl acetate | overnight |
| 20mg | Clear | 1M | ethyl acetate | overnight |

### Example 13 Preparation of crystal form B-1 to crystal form F and crystal form H-1, H-2 and H-3 of the compound of formula X

20mg of the free base of the compound of formula X (amorphous) was weighed into each sample bottle, and 1-3ml of the corresponding organic solvent was added, after the compound was dissolved, the corresponding acid was added according to the acid-base molar ratio of 1.2:1 (50µL for 1M acid, 100µL for 0.5M acid, 200µL for 0.25M acid) to conduct a salt-forming reaction, A magnetic stir bar was added and the solution was reacted for 4 hours under a 50°C water bath using a magnetic stirrer, the reaction was carried out while heating and stirring. After the reaction was completed, the reaction solution was cooled to room temperature, the samples in solution state were dried and sent for XRD test, the samples in turbid liquid state were centrifuged, dried and sent for XRD test. The X-ray powder diffraction patterns of crystal forms B-1, B-2, B-3, C, D, E-1, E-2, F, G, H-1, H-2 and H-3 are shown in Figure 2-1, Figure 2-2, Figure 2-3, Figure 3, Figure 4, Figure 5-1, Figure 5-2, Figure 6, Figure 7, Figure 8-1, Figure 8-2 and Figure 8-3 respectively. The results are shown in table 10:

**Table 10**

| Acid | Concentration (mol/L) | Volume (µl) | isopropanol | acetone | ethyl acetate | acetonitrile |
|---|---|---|---|---|---|---|
| sulfuric acid | 0.5 | 100 | crystal form B-1 | crystal form B-3 | crystal form B-2 | crystal form B-2 |
| hydrobromic acid | 1 | 50 | amorphous | crystal form C | crystal form C | crystal form C |
| phosphoric acid | 1 | 50 | amorphous | amorphous | crystal form D | amorphous |
| methanesulfonic acid | 1 | 50 | crystal form E-1 | crystal form E-2 | crystal form E-2 | crystal form E-1 |
| tartaric acid | 1 | 50 | amorphous | amorphous | crystal form F | amorphous |
| succinic acid | 0.5 | 100 | crystal form H-1 | crystal form H-3 | crystal form H-2 | crystal form H-1 |

### Example 14 Stability experiment

The compound of formula X (amorphous) and crystal form A are placed in a 40°C/75%RH accelerated stability box and a 60°C oven respectively, and take samples at fixed time points. The experimental conditions and results are shown in table 11:

**Table 11**

| Sample | Experimental Conditions RRT | Total Impurity % | Content % |
|---|---|---|---|
| the compound of formula X (amorphous) | 0 day | 0 | 100.00 |
| | 40°C-8 days | 0 | 100.62 |
| | 40°C-14 days | 0 | 100.70 |
| | 60°C-8 days | 0 | 100.82 |
| | 60°C-14 days | 0 | 100.81 |
| crystal form A | 0 day | 0.24 | 98.40 |
| | 40°C-8 days | 0.15 | 99.29 |
| | 40°C-14 days | 0.26 | 100.62 |
| | 60°C-8 days | 0.18 | 98.56 |
| | 60°C-14 days | 0.30 | 98.02 |

It can be seen from the above table that no increase of related substances was detected for the compound of formula X (amorphous) after an accelerated condition of 40°C/75% RH and a 60°C high temperature condition for 8 days and 14 days, there is no significant change in the content. The compound is stable.

There is no significant change in the content of crystal form A after an accelerated condition of 40°C/75% RH and a 60°C high temperature condition for 8 days and 14 days, the compound is relatively stable. The characteristic peaks of the XRD spectrum have no change, the crystal form is stable, and is the original crystal form.

### Example 15 Solubility experiment

About 5mg of the compound of formula X (amorphous), crystal form I and crystal form A were weighed into each centrifuge tube, and 0.5ml or 1ml of corresponding medium was added, and ultrasonic vibration is conducted to make it dissolved as much as possible to form a supersaturated solution, centrifuged and the supernatant (if the solubility is high, dilute it) was taken into the liquid chromatography to calculate the solubility, 5.69mg of the compound of formula X (amorphous) can be completely dissolved in 0.5ml of 0.1M HCl medium, 4.24mg of crystal form I can be completely dissolved in 1ml of 0.1M HCl medium, the experimental results are shown in table 12:

**Table 12**

| Sample | Peak area/state | Concentration mg/ml | Dilution ratio | Solubility mg/ml |
|---|---|---|---|---|
| Amorphous-0.1 M HCl | clear | ≥11.38 | NA | ≥11.38 |
| Amorphous- H₂O | 100.2 | 0.011 | NA | 0.011 |
| Amorphous-pH4.5 | 80.5 | 0.009 | NA | 0.009 |
| Amorphous-pH6.8 | 63.8 | 0.007 | NA | 0.007 |
| crystal form I-0.1 M HCl | clear | ≥4.24 | NA | ≥4.24 |
| crystal form I -H₂O | 67 | 0.007 | NA | 0.007 |
| crystal form I -pH4.5 | 71.7 | 0.008 | NA | 0.008 |
| crystal form I -pH6.8 | 55.9 | 0.006 | NA | 0.006 |
| crystal form A-0.1 M HCl | 956.9 | 0.123 | 20 | 2.459 |
| crystal form A-H₂O | 546.6 | 0.068 | 20 | 1.366 |
| crystal form A-pH4.5 | 1231.4 | 0.160 | NA | 0.160 |
| crystal form A-pH6.8 | 512.4 | 0.064 | NA | 0.064 |

It can be seen from the above table that the compound of formula X (amorphous) and crystal form I have higher solubility in 0.1M HCl medium, but the solubility is very low in water, pH4.5 and pH6.8 medium, the crystal form A has higher solubility in 0.1M HCl medium and water, but lower solubility in pH4.5 and pH6.8 medium.

### Example 16 Stability experiment of the crystal form of compound of formula X free base

80 mg of the compound of formula X in amorphous form, crystal form I, crystal form II and crystal form III were weighed respectively, and mixed them in a sample bottle; 20 mg of the mixture was weighed into each centrifuge tube, a total of 15 samples (5 experimental solvents, 3 experimental conditions); Experimental solvents: acetone, acetonitrile, ethyl acetate, isopropanol and ethanol; Experimental conditions: 1 day and 7 days at room temperature, 1 day at 50°C. The experimental conditions and results are as follows:
The XRD patterns show that the amorphous and three crystal forms all transform into crystal form I after mixing and shaking under suspension in acetone at 50°C for 1 day, and at room temperature for 1 day and 7 days, and crystal form I is a stable crystal form;

The XRD patterns show that the amorphous and three crystal forms all transform into crystal form I after mixing and shaking under suspension in acetonitrile at 50°C for 1 day, and at room temperature for 1 day and 7 days, and crystal form I is a stable crystal form;

The XRD patterns show that the amorphous and three crystal forms all transform into crystal form I after mixing and shaking under suspension in ethyl acetate at 50°C for 1 day, and at room temperature for 1 day and 7 days, and crystal form I is a stable crystal form;

The XRD patterns show that the amorphous and three crystal forms all transform into crystal form I after mixing and shaking under suspension in isopropanol at 50°C for 1 day, and at room temperature for 1 day and 7 days, and crystal form I is a stable crystal form;

The XRD patterns show that the amorphous and three crystal forms all transform into crystal form III after mixing and shaking under suspension in ethanol at 50°C for 1 day, and at room temperature for 1 day and 7 days. Since the amorphous form of the compound of formula X is transformed into crystal form III by mixing and shaking under suspension and cooling crystallization in ethanol, crystal form III is a stable crystal form in ethanol.

### Example 17 Stability experiment of the crystal form I

The sample is weighed and placed in an oven at 60°C, and taken for test at a fixed time point. The experimental results are shown in table 13:

**Table 13**

| Conditions | Content% | Relative substance% |
|---|---|---|
| Crystal form I/O day | 100.11 | none |
| 60°C-1 week | 99.91 | none |
| 60°C-2 weeks | 99.76 | none |

It can be seen from the above table that the content of crystal form I has not changed after 1 week and 2 weeks at 60°C, no related substances have been detected, and the chemical properties are stable; the XRD pattern shows that the crystal form has not changed, and has stable physical properties.

### Example 18 Pharmaceutical composition

Tablet of crystal form A is prepared from the following components:

| | |
|---|---|
| Crystal form A | 12g |
| starch | 43g |
| lactose | 35g |
| PVP | 6g |
| Carboxymethyl starch sodium | 4g |
| Sodium dodecyl sulfate | 0.8g |
| Magnesium stearate | 1.2g |

The crystal form A and starch are mixed and sieved, and then well mixed with the above other components, and tableted directly according to a conventional method.

### Example 19 Pharmaceutical composition

Capsule of crystal form I is prepared from the following components:

| | |
|---|---|
| Crystal form I | 18g |
| starch | 43g |
| lactose | 30g |
| PVP | 2.8g |
| Carboxymethyl starch sodium | 2.8g |
| Sodium dodecyl sulfate | 1.2g |
| Magnesium stearate | 1.2g |

The crystal form I and starch are mixed and sieved, then well mixed with the above other components, and filled into ordinary gelatin capsules according to a conventional method.

### Bioassay

### Test 1: Lantha screening kinase reaction experimental method

The compound was predissolved in 100% DMSO. 10 mM drug stock solution was obtained by dissolution at room temperature and then serially diluted with 8 vol% DMSO solution to a final concentration of 10-0.005 µM. 2.5 µl of a solution of substance to be tested and 2.5 µl of kinase (Invitrogen PV3363) diluted with the reaction buffer were added into each well of 384-well plate (Corning 3676), and then the mixture of Fluososcei-PolyGT (Invitrogen PV3610) substrate and ATP (Invitrogen PV3227) diluted with 5 µl of the reaction buffer were added to initiate the reaction. Among the wells, the kinase in the blank well was replaced with a reaction buffer and the kinase well (Enzyme) was not added with any drug. After shaking at 25 °C for 60 minutes in the dark, 10 µl of Detection Solution (mixture of Invitrogen PV3528 and EDTA, which was diluted with TR-FRET dilution buffer, the working concentration of EDTA was 5 mM, the working concentration of Lanthascreening Tb PY20 antibody was 0.2 nM) was added and shaken at room temperature for 30 minutes. The plates were read on a VictorX5 fluorescent plate reader (PerkinElmer) and the light absorption at an excitation wavelength of 340 nm and emission wavelengths of 500 nm and 520 nm was measured.

The calculation method for the inhibition ratio (referring to the specification of Invitrogen, PV3363) was as follows:
1. Emission ratio (ER): Coumarin Emission (520 nm)/Fluorescein Emission (500 nm)
2. The inhibition ratio (IR): (ERₖᵢₙₐₛₑ-ER_{test compound})/(ERₖᵢₙₐₛₑ-ER_{blank}) × 100%. The half-inhibitory concentration IC50 was calculated by fitting with XLFIT 5.0 software (IDBS, UK). The results were show in table 14:

**Table 14 the inhibition of compounds against BTK WT**

| Compound | BTK WT |
|---|---|
| | IC50(nM) |
| Compound of formula X | 2.4 |
| (S)-6-((1-acryloylpiperidin-3-yl)amino)-7-fluoro-4-((2-fluoro-4-morpholinophenyl)amino)-1,2-di hydrogen-3H-pyrrolo[3,4-c]pyridin-3-one | 48 |

Wherein the compound (S)-6-((1-acryloylpiperidin-3-yl)amino)-7-fluoro-4-((2-fluoro-4-morpholinophenyl)amino)-1,2-di hydrogen-3H-pyrrolo[3,4-c]pyridin-3-one was prepared with (S)-tert-butyl 3-aminopiperidine-1-carboxylate as a raw material according to the method of Example 1.

### Test 2: Experimental method for detecting HTRF by intracellular βBTK Y223 phosphorylation

The compound was predissolved in 100% DMSO. 10 mM drug stock solution was obtained by dissolution at room temperature and then serially diluted with 5 vol% DMSO solution to a final concentration of 3-0.0014 µM. Ramos cells were seeded into 96-well plates at a density of 4×10⁵/well with 45 µl of 1640 medium containing 10% (V/V) FBS per well, and 5 µl of diluted solution of substance to be tested was added to each well, which was incubated for 1 hour at 37 °C, 5% (V/V) CO₂. 10 µl of sodium pervanadate dilution (diluted with 1640 without serum) was added, and to the negative control wells was added 10 µl of serum-free medium, which was incubated at 25 °C for 30 minutes on a shaker. 20 µl of lysate (4x lysate : blocked mother liquor is 25:1) was added to each well and incubated for 30 minutes at 25 °C on a shaker. It was shaken on an oscillator at 800 rpm for 1 minute. 16 µl of cell lysate was added to a 384-well plate (Greiner 784075), and 4 µl of pre-mixed antibody solution (Phospho-BTK d2 antibody and Phospho-BTK Cryptate antibody diluted 20-fold with the test solution) was added, which was incubated at 25 °C overnight on a shaker. The plates were read on a VictorX5 fluorescent plate reader (PerkinElmer) and the light absorption at an excitation wavelength of 317 nm and emission wavelengths of 500 nm and 520 nm was measured (referring to the specification of 63ADK017PEH, Cisbio). The half-inhibitory concentration IC50 was calculated by fitting with XLFIT 5.0 software (IDBS, UK). The results were show in table 15:

**Table 15 Inhibition activity of compound against βBTK Y223 cell**

| Compound | βBTK Y223 |
|---|---|
| | IC50(nM) |
| Compound of formula X | 3 |

It can be seen from tables 14 and 15 that the compound of formula X has higher inhibitory activity against enzymes and cells.

### Test 3: Wild type EGFR kinase inhibition test

All the following reagents used in z-lyte test are commercially available from Invitrogen.

The inhibitory effects of compounds to be tested on wild-type EGFR kinase (Invitrogen, PV3872) were measured by z-lyte methods.

The working concentration of each component in 10 µl wild-type EGFR kinase reaction system was: 10 µM ATP, 0.8 ng/µl wild-type EGFR kinase (Invitrogen, PV3872), 2 µM Tyr04 substrate (Invitrogen, PV3193). After the compounds to be tested were added, the final concentration of DMSO was 2%.

10 mM drug stock solutions dissolved at room temperature were gradiently diluted with 4 % DMSO in water to a final concentrations of 10-0.005 µM. To each well were added 2.5 µl of a solution of the compounds to be tested and 5 µl of a mixture of wild-type EGFR kinase and Tyr04 substrate diluted with a reaction buffer, and then 2.5 µl of ATP was added to initiate the reaction. Reaction buffer instead of ATP were added to C1 wells, no drugs were added to C2 wells, and the phosphorylated substrates were added to C3 wells according to the instruction. After shaking on a shaker at 25 °C for 60 minutes in the dark, 5 µl of Development Reagent B (Invitrogen, diluted with TR-FRET dilution buffer) was added, and reacted on a shaking table at room temperature for 60 min. The plates were read in a VictorX5 fluorescent microplate reader (PerkinElmer) and the light absorbance at an excitation wavelength of 405 nm and emission wavelengths of 450 nm and 520 nm was measured (For example, C3₅₂₀ₙₘ represents the reading of C3 well at 520 nm).

The calculation method for the inhibition ratio (referring to the specification of Invitrogen, PV3363) was as follows:
1. ER=Coumarin Emission (450 nm)/Fluorescein Emission (520 nm)
2. Phosphorylation ratio= (1-((ER×C3₅₂₀ₙₘ-C3₄₅₀ₙₘ)/((C1₄₅₀ₙₘ-C3₄₅₀ₙₘ)+ER×(C3₅₂₀ₙₘ -C1 ₅₀ₙₘ)))) ×100%
3. Inhibition ratio (IR) = (1- phosphorylation ratio of the test compound) / (phosphorylation ratio of C2)) × 100%

The half-inhibitory concentration IC₅₀ was calculated by fitting with XLFIT 5.0 software (IDBS, UK).

**Table 16 Inhibition activity of compound against to EGFR WT**

| Compound | EGFR WT |
|---|---|
| | IC50(nM) |
| Compound of formula X | 932 |

It can be seen from table 16 that the compound of formula X has lower inhibitory activity against wild-type EGFR kinase. Therefore, the compound of formula X has selective inhibitory activities against BTK WT kinase.

All publications mentioned herein are incorporated by reference as if each individual document is cited as a reference, as in the present application. It should also be understood that, after reading the above teachings of the present disclosure, those skilled in the art can make various changes or modifications, equivalents of which fall in the scope of claims as defined in the appended claims.

## Claims

1. A compound of formula X, or a pharmaceutically acceptable salt thereof,

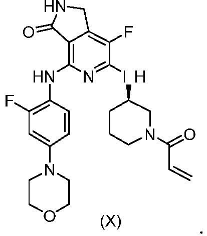

2. The compound of formula X or the pharmaceutically acceptable salt thereof according to claim 1, wherein the pharmaceutically acceptable salt is selected from the group consisting of hydrochloride, sulfate, hydrobromide, phosphate, methanesulfonate, maleate, L-tartrate, citrate, fumarate and succinate.

3. A polymorph of the compound of formula X or a polymorph of a pharmaceutically acceptable salt of the compound of formula X, 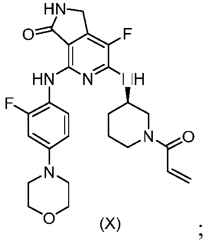 wherein the pharmaceutically acceptable salt is selected from the group consisting of hydrochloride, sulfate, hydrobromide, phosphate, methanesulfonate, maleate, L-tartrate, citrate, fumarate and succinate.

4. The polymorph according to claim 3, wherein the polymorph is selected from the following group consisting of:
A crystalline form of the hydrochloride of the compound of formula X, i.e. crystal form A, a X-ray powder diffraction pattern of which has peaks at diffraction angles 2θ(°) values of the following group A-1: 14.75±0.2, 15.97±0.2, 17.20±0.2, 18.94±0.2, 19.72±0.2, 22.15±0.2, 24.35±0.2, 25.12±0.2, 26.21±0.2, and 26.80±0.2;
B-1 crystalline form of the sulfate of the compound of formula X, i.e. crystal form B-1, a X-ray powder diffraction pattern of which has peaks at diffraction angles 2θ(°) values of the following group B-1-1: 10.27±0.2, 14.06±0.2, 14.41±0.2, 17.59±0.2, 19.39±0.2, 21.84±0.2, 26.38±0.2, and 26.68±0.2;
B-2 crystalline form of the sulfate of the compound of formula X, i.e. crystal form B-2, a X-ray powder diffraction pattern of which has peaks at diffraction angles 2θ(°) values of the following group B-2-1: 8.59±0.2, 10.64±0.2, 13.90±0.2, 14.38±0.2, 15.53±0.2, 17.05±0.2, 17.26±0.2, 17.75±0.2, 19.28±0.2, 21.85±0.2, 25.82±0.2, 26.32±0.2, and 26.62±0.2;
B-3 crystalline form of the sulfate of the compound of formula X, i.e. crystal form B-3, a X-ray powder diffraction pattern of which has peaks at diffraction angles 2θ(°) values of the following group B-3-1: 8.59±0.2, 10.21±0.2, 10.60±0.2, 11.39±0.2, 13.03±0.2, 13.93±0.2, 14.38±0.2, 15.49±0.2, 15.82±0.2, 17.03±0.2, 17.71±0.2, 19.30±0.2, 20.23±0.2, 21.59±0.2, 21.97±0.2, 23.95±0.2, 24.62±0.2, 26.23±0.2, and 26.65±0.2;
C crystalline form of the hydrobromide of the compound of formula X, i.e. crystal form C, a X-ray powder diffraction pattern of which has peaks at diffraction angles 2θ(°) values of the following group C-1: 15.26±0.2, 15.91±0.2, 17.09±0.2, 18.43±0.2, 18.76±0.2, 19.49±0.2, 20.47±0.2, 21.91±0.2, 24.10±0.2, 24.88±0.2, 25.87±0.2, and 26.48±0.2;
D crystalline form of the phosphate of the compound of formula X, i.e. crystal form D, a X-ray powder diffraction pattern of which has peaks at diffraction angles 2θ(°) values of the following group D-1: 12.24±0.2, 13.93±0.2, 17.24±0.2, 18.18±0.2, 23.93±0.2, 26.38±0.2, and 26.68±0.2;
E-1 crystalline form of the methanesulfonate of the compound of formula X, i.e. crystal form E-1, a X-ray powder diffraction pattern of which has peaks at diffraction angles 2θ(°) values of the following group E-1-1: 8.56±0.2, 11.39±0.2, 17.47±0.2, 17.80±0.2, and 26.32±0.2;
E-2 crystalline form of the methanesulfonate of the compound of formula X, i.e. crystal form E-2, a X-ray powder diffraction pattern of which has peaks at diffraction angles 2θ(°) values of the following group E-2-1: 15.79±0.2, 16.76±0.2, 17.41±0.2, 17.80±0.2, 20.26±0.2, 21.05±0.2, 24.10±0.2, 25.63±0.2, 26.53±0.2, 26.92±0.2, and 27.50±0.2;
F crystalline form of the tartrate of the compound of formula X, i.e. crystal form F, a X-ray powder diffraction pattern of which has peaks at diffraction angles 2θ(°) values of the following group F-1: 18.58±0.2, 19.84±0.2, 20.56±0.2, 24.88±0.2, 28.73±0.2, 29.45±0.2, 31.81±0.2, and 33.28±0.2;
G crystalline form of the fumarate of the compound of formula X, i.e. crystal form G, a X-ray powder diffraction pattern of which has peaks at diffraction angles 2θ(°) values of the following group G-1: 16.06±0.2, 18.76±0.2, 20.32±0.2, 21.49±0.2, 22.52±0.2, 22.84±0.2, 24.32±0.2, 24.50±0.2, 26.06±0.2, and 28.48±0.2;
H-1 crystalline form of the succinate of the compound of formula X, i.e. crystal form H-1, a X-ray powder diffraction pattern of which has peaks at diffraction angles 2θ(°) values of the following group H-1-1: 21.70±0.2;
H-2 crystalline form of the succinate of the compound of formula X, i.e. crystal form H-2, a X-ray powder diffraction pattern of which has peaks at diffraction angles 2θ(°) values of the following group H-2-1: 19.78±0.2, 21.63±0.2, 25.96±0.2, and 31.23±0.2; or
H-3 crystalline form of the succinate of the compound of formula X, i.e. crystal form H-3, a X-ray powder diffraction pattern of which has peaks at diffraction angles 2θ(°) values of the following group H-3-1: 12.20±0.2, 19.72±0.2, 19.84±0.2, 25.82±0.2, and 31.21±0.2.

5. The polymorph according to claim 3, wherein the polymorph is selected from the following group consisting of:
crystal form I of the compound of formula X, a X-ray powder diffraction pattern of which has peaks at diffraction angles 2θ(°) values of the following group I-1: 16.01±0.2, 18.64±0.2, 20.27±0.2, 21.40±0.2, 22.84±0.2, and 24.49±0.2;
crystal form II of the compound of formula X, a X-ray powder diffraction pattern of which has peaks at diffraction angles 2θ(°) values of the group II-1: 7.32±0.2, 9.84±0.2, 13.56±0.2, 17.47±0.2, 22.73±0.2, 24.37±0.2, and 25.09±0.2; and
crystal form III of the compound of formula X, a X-ray powder diffraction pattern of which has peaks at diffraction angles 2θ(°) values of the group III-1: 9.52±0.2, 11.77±0.2, 12.43±0.2, 12.78±0.2, 15.31±0.2, 16.33±0.2, 16.84±0.2, 17.83±0.2, 18.49±0.2, 19.57±0.2, 20.15±0.2, 21.71±0.2, 23.26±0.2, 23.84±0.2, 24.52±0.2, and 25.30±0.2.

6. The polymorph according to claim 4, wherein
the X-ray powder diffraction pattern of the crystal form A is substantially as **characterized in** Figure 1-1;
the X-ray powder diffraction pattern of the crystal form B-1 is substantially as **characterized in** Figure 2-1;
the X-ray powder diffraction pattern of the crystal form B-2 is substantially as **characterized in** Figure 2-2;
the X-ray powder diffraction pattern of the crystal form B-3 is substantially as **characterized in** Figure 2-3;
the X-ray powder diffraction pattern of the crystal form C is substantially as **characterized in** Figure 3;
the X-ray powder diffraction pattern of the crystal form D is substantially as **characterized in** Figure 4;
the X-ray powder diffraction pattern of the crystal form E-1 is substantially as **characterized in** Figure 5-1;
the X-ray powder diffraction pattern of the crystal form E-2 is substantially as **characterized in** Figure 5-2;
the X-ray powder diffraction pattern of the crystal form F is substantially as **characterized in** Figure 6;
the X-ray powder diffraction pattern of the crystal form G is substantially as **characterized in** Figure 7;
the X-ray powder diffraction pattern of the crystal form H-1 is substantially as **characterized in** Figure 8-1;
the X-ray powder diffraction pattern of the crystal form H-2 is substantially as **characterized in** Figure 8-2;
the X-ray powder diffraction pattern of the crystal form H-3 is substantially as **characterized in** Figure 8-3.

7. The polymorph according to claim 5, wherein
the X-ray powder diffraction pattern of the crystal form I is substantially as **characterized in** Figure 9-1;
the X-ray powder diffraction pattern of the crystal form II is substantially as **characterized in** Figure 10;
the X-ray powder diffraction pattern of the crystal form III is substantially as **characterized in** Figure 11.

8. A method for preparing the crystal form I of the compound of formula X, wherein the compound of formula X is as follows: 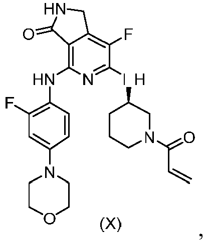 the method comprises the following steps:
(a) suspending the compound of formula X in a solvent at 10°C-60°C, wherein the solvent is water, acetonitrile, isopropanol, acetone, ethyl acetate, tetrahydrofuran, n-heptane or methyl tert-butyl ether; and
(b) mixing and centrifuging under suspension the mixture of step (a), or mixing and shaking under suspension the mixture of step (a) and separating, to obtain the crystal form I.

9. A method for preparing the crystal form I of the compound of formula X, wherein the compound of formula X is as follows: 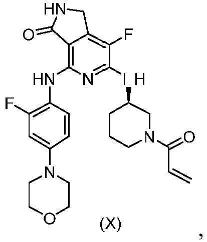 the method comprises the following steps:
(i) dissolving the compound of formula X in a solvent at 30°C-60°C; wherein the solvent is isopropanol, acetone or tetrahydrofuran; and
(ii) cooling and crystallizing the mixture of step (i), and then separating to obtain the crystal form I.

10. A pharmaceutical composition, wherein the pharmaceutical composition includes:
(a) the compound of formula X, or the pharmaceutically acceptable salt thereof according to claim 1; and (b) a pharmaceutically acceptable carrier.

11. A pharmaceutical composition, wherein the pharmaceutical composition includes:
(a) the polymorph according to claim 3; and (b) a pharmaceutically acceptable carrier.

12. Use of the compound of formula X, or the pharmaceutically acceptable salt thereof according to claim 1, or the pharmaceutical composition according to claim 10 in the preparation of a drug for the treatment and/or prevention of tumors, cancers, proliferative diseases, allergic diseases, autoimmune diseases or inflammatory diseases.

13. Use of the polymorph of the compound of formula X, or the polymorph of pharmaceutically acceptable salt of the compound of formula X according to claim 3, or the pharmaceutical composition according to claim 11 in the preparation of a drug for the treatment and/or prevention of tumors, cancers, proliferative diseases, allergic diseases, autoimmune diseases or inflammatory diseases.
